(19) Europäisches Patentamt
European Patent Office
Office européen des brevets

(11) EP 2 332 930 A1

(12) EUROPEAN PATENT APPLICATION

(43) Date of publication:
**15.06.2011 Bulletin 2011/24**

(51) Int Cl.:
***C07D 333/16*** *(2006.01)* ***A61K 31/381*** *(2006.01)*
***A61P 29/00*** *(2006.01)*

(21) Application number: **09384006.4**

(22) Date of filing: **23.11.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicant: **Laboratorios del. Dr. Esteve, S.A.**
**08041 Barcelona (ES)**

(72) Inventors:
- **Buschmann, Helmut Heinrich**
  **52076 Aachen (Walheim) (DE)**
- **Benet Buchholz, Jordi**
  **43893 Altafulla (Tarragona) (ES)**
- **Ceron Bertran, Jordi Carles**
  **43005 Tarragona (ES)**
- **Freixas Pascual, Gloria**
  **08770 St. Sadurni d'Anoia (Barcelona) (ES)**

(74) Representative: **Peters, Hajo et al**
**ZACCO GmbH**
**Bayerstrasse 83**
**80335 München (DE)**

(54) **Salts of duloxetine and NSAIDs for the treatment of pain**

(57) The present invention relates to salts of duloxetine and NSAIDs, processes for preparation of the same and their uses as medicaments or in pharmaceutical formulations, more particularly for the treatment of pain.

EP 2 332 930 A1

## Description

The present invention relates to salts of duloxetine and NSAIDs, processes for preparation of the same and their uses as medicaments or in pharmaceutical formulations, more particularly for the treatment of pain.

Pain is a complex response that has been functionally categorized into sensory, autonomic, motor, and affective components. The sensory aspect includes information about stimulus location and intensity while the adaptive component may be considered to be the activation of endogenous pain modulation and motor planning for escape responses. The affective component appears to include evaluation of pain unpleasantness and stimulus threat as well as negative emotions triggered by memory and context of the painful stimulus.

In general, pain conditions can be divided into chronic and acute. Chronic pain includes neuropathic pain and chronic inflammatory pain, for example arthritis, or pain of unknown origin, as fibromyalgia. Acute pain usually follows non-neural tissue injury, for example tissue damage from surgery or inflammation, or migraine.

(+)-(*S*)-N-Methyl-N-[3-(naphthalene-1-yloxy)-3-(2-thienyl)-propyl]amine, having the INN-name duloxetine and being also described as (+)-(*S*)-duloxetine or (*S*)-duloxetine is known to be a potent serotonin and norepinephrine reuptake inhibitor (SNRI). Duloxetine is marketed for the treatment of a variety of diseases like anxiety and depression but also including pain, especially diabetic peripheral neuropathy.

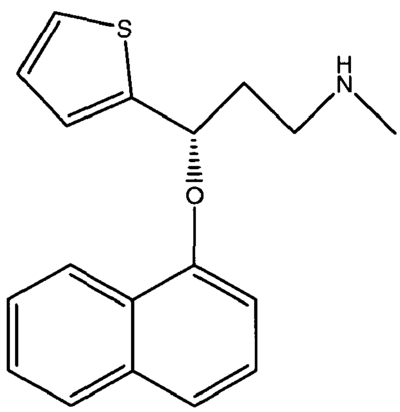

Duloxetine/(S)-Duloxetine

Duloxetine is a commonly used drug. The usual application route is orally with capsules containg duloxetine hydrochloride in enterically coated pellets being applied with doses of 20, 30 and 60 mg of active duloxetine. The enteric coating is necessary as duloxetine is acid labile and therefore would have been in danger to become degenerated in the acid environment of the stomach. The main side effects, nausea, somnolence, insomnia and dizzines, are occuring in 10 to 20 % of the patients. In addition a number of severe side-effects have been reported. Being - as said above - a wildly used drug there is a continuous need to improve its properties to achieve a number of effects like improved formulations, higher efficacy, reduction of side effects e.g. through reduction of the necessary dosis etc.

Thus it was the objective of the current invention to provide new means of improving the properties of duloxetine, especially in regard to the treatment of pain, by providing new drugable forms of duloxetine.

Especially desirable improvements/advantages of the new drugable form would include:

- improvement of physicochemical properties in order to facilitate the formulation, the manufacture, or to enhance the absorption and/or the bioavailability;
- being more active when compared to duloxetine or its hydrochloride salt alone;
- providing a form of a salt having a beneficial pharmacological effect in itself, thus allowing for a highly efficient dose/ weight relation of the final active principle;
- allowing the use of a lower therapeutic dose;
- having a synergistic effect;
- being easily obtainable, easy to manufacture;
- allowing more flexibility in formulating, or facilitating its formulation,
- being highly soluble, thus allowing better dissolution rates, especially if dissolving in an aqueous physiological surrounding;
- improving stability;

- allowing new routes of administration;
  or finally
- minimizing/reducing the side effects, especially the severe side effects, assigned to duloxetine.

Most desirably the new drugable forms should combine more than one, most of these advantages.
This objective was achieved by providing new salts of duloxetine with NSAIDs. It was found that duloxetine was able to form salts with active agents, especially with active agents having analgesic activity.
These salts show improved properties if compared to any of the active principles alone. This association of the two active principles into the same salt exhibits several advantages. Being linked as ion and counter-ion, they behave as a single chemical entity, thus facilitating the use of duloxetine and the NSAID in certain formulation, allowing a reduced dosage and maybe opening new treatments not being followed before in the art.
By that way the physico-chemical properties are improved. The formulation of the association is even easier with a solid to manipulate and an enhanced stability. The solubility may be also enhanced.
Another advantage is that the combination of the two active principles into one unique species seems to allow for a better Pharmacokinetic/Pharmacodynamic (PKPD) which helps in the treatment of pain.
"NSAID" is defined by the basis of its activity being inhibition of cyclooxygenase (COX), one of the two activities of prostaglandine endoperoxide synthase (PGHS). PGHS is a key enzyme in the prostaglandin pathway. NSAIDs have analgesic activity in a number of pain symptoms, with acetyl salicylic acid known under its trademark aspirin - despite being more than 100 years old - being an outstandingly used pharmaceutical. Besides aspirin other NSAIDs whose use generally is also centered on anti-inflammatory action like ibuprofen, naproxen or diclofenac are among the worldwide most frequently applied pharmaceutical compounds. Some preferred NSAIDs in the meaning of this application are those with a functional group showing an acid function like carboxylic acids, sulfonic acids etc. Examples include salicylates, anthranilates, arylacetic acids/arylalkanoic acids, arylpropionic acids and others as oxicams as well as ketorolac, fenbufen, sulindac or nimesulide.
In one preferred embodiment of the salt according to the invention the NSAID is selected from salicylates, anthranilates, arylacetic acids/arylalkanoic acids, oxicams and arylpropionic acids as well as from ketorolac, fenbufen and nimesulide
Examples of salicylates are: Acetylsalicylic acid, Diflunisal, Ethenzamide, Salicylamide, Triflusal, Fosfosal, and Benorylate.
Examples of anthranilates are: Etofenamate, Flufenamic acid, Meclofenamic acid, Mefenamic acid, Niflumic acid, and Tolfenamic acid.
Examples of arylacetic acids/arylalkanoic acids are: Acemetacin, Oxametacin, Glucametacin, Proglumetacin, Bufexamac, Diclofenac, Alcofenac, Aceclofenac, Indomethacin, Lonazolac, Sulindac, Tolmetin, Amtolmetin guacil, Mofezolac, Bromfenac, Ketorolac, Nabumetone, Fentiazac, Fenbufen and Felbinac.
Examples of arylpropionic acids are: Flurbiprofen, Flurbiprofen axetil, Ibuprofen, Ketoprofen, Naproxen, Tiaprofenic acid, Zaltoprofen, Pirprofen, Fenoprofen, Vedaprofen, Nepafenac, Amfenac, and Clidanac.
Examples of oxicams are: piroxicam, isoxicam, lornoxicam, tenoxicam, droxicam and meloxicam.
In another preferred embodiment of the salt according to the invention the NSAID is selected from

Acetylsalicylic acid, Diflunisal, Ethenzamide, Salicylamide, Triflusal, Fosfosal, Benorylate, Paracetamol, Propacetamol, Phenidine, Etofenamate, Flufenamic acid, Meclofenamic acid, Mefenamic acid, Niflumic acid, Tolfenamic acid, Acemetacin, Oxametacin, Glucametacin, Proglumetacin, Bufexamac, Diclofenac, Alcofenac, Aceclofenac, Indomethacin, Lonazolac, Sulindac, Tolmetin, Amtolmetin guacil, Mofezolac, Bromfenac, Nabumetone, Fentiazac, Felbinac, Flurbiprofen, Flurbiprofen axetil, Ibuprofen, Ketoprofen, Naproxen, Tiaprofenic acid, Zaltoprofen, Pirprofen, Fenoprofen, Vedaprofen, Nepafenac, Amfenac, Clidanac, Metamizol, Propylphenazone, Kebuzone, Mofebutazone, Oxyphenbutazone, Phenylbutazone, Apazone, Isoxicam, Lornoxicam, Piroxicam, Tenoxicam, Ketorolac, Proquazone, Oxaprozine, Ditazole, Etodolac, Meloxicam, Nimesulide, Celecoxib, Etoricoxib, Lumiracoxib, Parecoxib, Rofecoxib, Valdecoxib, Cimicoxib, Bermoprofen, Pelubiprofen, Tenosal, Aceneuramic acid, Pirazolac, Xinoprofen, Flobufen, Anirolac, Zoliprofen, Bromfenac, Pemedolac, Dexpemedolac, Bindarit, Romazarit, and Fenbufen; or their stereoisomers or solvates.

In another preferred embodiment of the salt according to the invention the NSAID is selected from

Acetylsalicylic Acid; Triflusal; Diflunisal; Meclofenamic acid; Mefenamic acid; Niflumic acid; Flufenamic acid; Diclofenac; Lonazolac; Acemetacin; Indomethacin; Tolmetin; Sulindac; Etodolac; Ketorolac; Flurbiprofen; Ibuprofen; Ketoprofen; Bermoprofen; Pelubiprofen; Tenosal; Aceneuramic acid; Pirazolac; Xinoprofen; Flobufen; Anirolac; Zoliprofen; Bromfenac; Pemedolac; Dexpemedolac; Bindarit; Romazarit; Naproxen; Tiaprofenic acid; Ketorolac; Fenbufen; Fenoprofen; Nimesulide; Piroxicam; Flobufen; and Oxaprozin, or their stereoisomers or solvates.

In another preferred embodiment of the salt according to the invention the NSAID is selected from acemetacin, fenbufen, ketorolac, nimesulide, piroxicam, sulindac, diclofenac; or their stereoisomers or solvates.

In general, all of these NSAIDs which have at least one stereogenic center are to be understood as being included herein in their racemic form or as diastereoisomers or enantiomers or mixtures thereof.

In a highly preferred embodiment the salt according to the invention is selected from

- Acemetacin/(*S*)-duloxetine amorphous salt;
- Fenbufen/(*S*)-duloxetine amorphous salt;
- Ketorolac/(*S*)-duloxetine amorphous salt;
- Nimesulide/(*S*)-duloxetine amorphous salt;
- Piroxicam/(*S*)-duloxetine amorphous salt;
- Sulindac/(*S*)-duloxetine amorphous salt;
- Diisopropyl ether hemisolvate salt of diclofenac/(*S*)-duloxetine;
- Isopropanol hemisolvate salt of diclofenac/(*S*)-duloxetine;
- Toluene hemisolvate salt of diclofenac/(*S*)-duloxetine;
- Acetone hemisolvate salt of diclofenac/(*S*)-duloxetine;
- Methyl ethyl ketone hemisolvate salt of diclofenac/(*S*)-duloxetine;
- Ethanol solvate of diclofenac/(*S*)-duloxetine salt; and
- Non-solvated diclofenac/(*S*)-duloxetine salt;

or from

- Acemetacin/(*S*)-duloxetine amorphous salt;
- Fenbufen/(*S*)-duloxetine amorphous salt;
- Ketorolac/(*S*)-duloxetine amorphous salt;
- Nimesulide/(*S*)-duloxetine amorphous salt;
- Piroxicam/(*S*)-duloxetine amorphous salt;
- Sulindac/(*S*)-duloxetine amorphous salt;
- Diisopropyl ether hemisolvate salt of diclofenac/(*S*)-duloxetine;
- Isopropanol hemisolvate salt of diclofenac/(*S*)-duloxetine;
- Toluene hemisolvate salt of diclofenac/(*S*)-duloxetine;
- Acetone hemisolvate salt of diclofenac/(*S*)-duloxetine;
- Methyl ethyl ketone hemisolvate salt of diclofenac/(*S*)-duloxetine;
- Ethanol solvate of diclofenac/(*S*)-duloxetine salt; and

- Non-solvated diclofenac/(*S*)-duloxetine salt.

In another preferred embodiment of the salt according to the invention the diisopropyl ether hemisolvate salt of diclofenac/(*S*)-duloxetine shows an X-Ray powder diffraction pattern with peaks [2θ] at 5.5, 8.6, 8.9, 9.4, 10.1, 11.0, 11.2, 11.9, 12.1, 12.3, 12.7, 13.3, 13.9, 14.1, 14.7, 15.2, 15.5, 16.5, 17.0, 17.3, 17.5, 17.6, 18.5, 19.1, 19.3, 19.5, 19.7, 19.9, 20.2 and 20.3 [˚] with the 2θ values being obtained using copper radiation ($CU_{K\alpha 1}$ 1.54060Å).
In a further preferred embodiment of the salt according to the invention the diisopropyl ether hemisolvate salt of diclofenac/(*S*)-duloxetine shows an endothermic peak corresponding to the melting point with an onset at 83˚C.
In another preferred embodiment of the salt according to the invention the isopropanol hemisolvate salt of diclofenac/(*S*)-duloxetine shows an X-Ray powder diffraction pattern with peaks [2θ] at 5.5, 9.0, 9.8, 11.0, 11.1, 12.1, 13.0, 13.7, 13.8, 16.3, 17.0, 17.2, 18.0, 18.4, 18.6, 19.2, 19.7, 19.8, 20.2, 21.4, 22.1, 23.6, 23.8, 24.4, 24.7, 25.0, 25.2, 25.4, 26.2, and 26.6 [˚] with the 2θ values being obtained using copper radiation ($Cu_{K\alpha 1}$ 1.54060Å).
In a further preferred embodiment of the salt according to the invention the isopropanol hemisolvate salt of diclofenac/(*S*)-duloxetine shows an endothermic peak corresponding to the melting point with an onset at 73˚C.
In another preferred embodiment of the salt according to the invention the toluene hemisolvate salt of diclofenac/(*S*)-duloxetine shows an X-Ray powder diffraction pattern with peaks [2θ] at 5.2, 9.0, 9.8, 10.4, 12.3, 12.6, 13.3, 14.0, 14.9, 16.5, 17.4, 17.8, 18.2, 18.5, 18.9, 19.1, 19.7, 20.0, 20.1, 20.7, 21.0, 21.4, 21.5, 22.6, 22.9, 23.1, 23.5, 24.3, 24.5, and 25.4 [˚] with the 2θ values being obtained using copper radiation ($Cu_{K\alpha 1}$ 1.54060Å).
In a further preferred embodiment of the salt according to the invention the toluene hemisolvate salt of diclofenac/(*S*)-duloxetine shows an endothermic peak corresponding to the melting point with an onset at 78˚C.
In another preferred embodiment of the salt according to the invention the acetone hemisolvate salt of diclofenac/(*S*)-duloxetine shows an X-Ray powder diffraction pattern with peaks [2θ] at 5.5, 9.0, 9.8, 11.0, 12.1, 12.9, 13.7, 15.8, 16.3, 17.1, 17.2, 18.1, 18.3, 18.5, 18.7, 19.2, 19.4, 19.8, 20.1, 21.3, 21.8, 22.2, 23.7, 24.4, 24.7, 25.2, 25.5, 26.1, and 26.2 [˚] with the 2θ values being obtained using copper radiation ($Cu_{K\alpha 1}$ 1.54060Å).
In a further preferred embodiment of the salt according to the invention the acetone hemisolvate salt of diclofenac/(*S*)-duloxetine shows an endothermic peak corresponding to the melting point with an onset at 101˚C.
In another preferred embodiment of the salt according to the invention the methyl ethyl ketone hemisolvate salt of diclofenac/(*S*)-duloxetine shows an X-Ray powder diffraction pattern with peaks [2θ] at 5.3, 5.8, 9.1, 9.6, 10.6, 12.3, 12.6, 13.4, 13.7, 14.0, 16.4, 16.8, 17.3, 18.1, 18.4, 18.8, 19.1, 19.4, 19.9, 20.1, 20.6, 20.9, 21.5, 21.9, 22.4, 22.8, 24.6, 25.4, 25.6, and 26,4 [˚] with the 2θ values being obtained using copper radiation ($Cu_{K\alpha 1}$ 1.54060Å).
In a further preferred embodiment of the salt according to the invention the methyl ethyl ketone salt of diclofenac/(*S*)-duloxetine shows an endothermic peak corresponding to the melting point with an onset at 80˚C.
In a further preferred embodiment of the salt according to the invention the single crystal of the ethanol solvate of diclofenac/(S)-duloxetine salt according to the invention has a monoclinic unit cell with the following dimensions:

$$a = 9.4952(5)\ \text{Å}$$

$$b = 10.6992(5)\ \text{Å}$$

$$c = 15.7550(9)\ \text{Å}$$

$$\alpha = 90.00°$$

$$\beta = 96.490(2)°$$

$$\gamma = 90.00°.$$

In another preferred embodiment of the salt according to the invention the non-solvated diclofenac/(*S*)-duloxetine salt shows an X-Ray powder diffraction pattern with peaks [2θ] at 5.6, 9.2, 9.9, 11.2, 12.4, 12.7, 13.2, 13.9, 16.2, 16.4, 17.3, 18.4, 18.8, 19.5, 20.0, 20.3, 20.6, 21.6, 22.6, 24.1, 24.9, 25.4, 26.0, 26.6, 27.2, 27.7, 29.2, 32.0, 32.6 and 33.7 [°] with the 2θ values being obtained using copper radiation ($Cu_{K\alpha 1}$ 1.54060Å).

In a further preferred embodiment of the salt according to the invention the non-solvated diclofenac/(*S*)-duloxetine salt shows an endothermic peak corresponding to the melting point with an onset at 75°C.

Another aspect of the present invention relates to a process for the production of a salt according to the invention as described above comprising the steps of:

a) dissolving or suspending the NSAID in organic solvent 1;
b) either before or after step (a) dissolving duloxetine either as a free base or as a salt in organic solvent 2,
c) mixing the solutions from step (a) and (b);
d) evaporating the solvent(s) of the mixture from step (c) to dryness,
e) optionally dissolving the residue of step (d) in organic solvent 3:
f) optionally removing the organic solvent 3;

Preferably in the process above

- the organic solvent 1 is selected from tetrahydrofuran or methanol or mixtures thereof; or from dichloromethane, and diisopropyl ether; and/or
- the organic solvent 2 is selected from methanol, and diisopropyl ether; and/or
- the organic solvent 3 is selected from diisopropyl ether, ethanol/water (1:1), or isopropanol, toluene, acetone, and methyl ethyl ketone.

Both parts of the salt are well-known drugs used for a long time worldwide. Due to the therapeutic interest in duloxetine in the treatment of pain symptoms like diabetic neuropathy and the well-known properties of NSAIDs in this field of medical indication, a further object of the present invention is a medicament containing a salt according to the invention.

Thus the invention also concerns a medicament comprising at least one salt according to the invention as described above and optionally one or more pharmaceutically acceptable excipients.

A further object of the invention is a pharmaceutical composition characterized in that it comprises an efficient amount of the salt according to the invention as described above, in a physiologically acceptable medium.

The medicament or pharmaceutical composition according to the present invention may be in any form suitable for the application to humans and/or animals, preferably humans including infants, children and adults and can be produced by standard procedures known to those skilled in the art. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament or pharmaceutical composition of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments or pharmaceutical compositions according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or modified or controlled release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts,

Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments or pharmaceutical compositions according to the present invention may also comprise an enteric coating, so that their dissolution is dependent on pH-value. Due to said coating the medicament may pass the stomach undissolved and the respective salts or their respective crystalline form is liberated in the intestinal tract. Preferably the enteric coating is soluble at a pH value of 5 to 7.5. Suitable materials and methods for the preparation are known from the prior art.

Typically, the medicaments or pharmaceutical compositions according to the present invention may contain 1-60 % by weight of one or more of the salts according to the invention as defined herein and 40-99 % by weight of one or more auxiliary substances (additives/excipients).

The medicaments or pharmaceutical compositions of the present invention may also be administered topically or via a suppository.

The daily dosage for humans and animals may vary depending on factors that have their basis in the respective species or other factors, such as age, sex, weight or degree of illness and so forth. The daily dosage for humans preferably is in the range of 10 to 2000 milligrams of active substance to be administered during one or several intakes per day.

A further aspect of the invention relates to a medicament or pharmaceutical composition according to the invention for the treatment of pain, preferably acute pain, chronic pain, neuropathic pain, hyperalgesia, allodynia or cancer pain, including diabetic neuropathy or diabetic peripheral neuropathy and osteoarthritis or fibromyalgia

A further aspect of the invention relates to the use of a salt according to the invention as described above for the treatment of pain, preferably acute pain, chronic pain, neuropathic pain, hyperalgesia, allodynia or cancer pain, including diabetic neuropathy or diabetic peripheral neuropathy and osteoarthritis or fibromyalgia.

A related further aspect of the invention is aimed at the use of a salt according to the invention as described above in the manufacture of a medicament for the treatment of pain, preferably acute pain, chronic pain, neuropathic pain, hyperalgesia, allodynia or cancer pain, including diabetic neuropathy or diabetic peripheral neuropathy and osteoarthritis or fibromyalgia. Preferably this use is provided for in form of a medicament or a pharmaceutical composition according to the invention as described above.

Another object of the current invention is a method for the treatment of pain, preferably acute pain, chronic pain, neuropathic pain, hyperalgesia, allodynia or cancer pain, including diabetic neuropathy or diabetic peripheral neuropathy and osteoarthritis or fibromyalgia, by providing to a patient in need thereof a sufficient amount of a salt according to the invention as described above. Preferably a salt according to the invention is provided in physiologically suitable form like e.g. in form of a medicament or a pharmaceutical composition according to the invention as described above.

The present invention is illustrated below with the help of the following figures and examples. These illustrations are given solely by way of example and do not limit the invention.

***Brief description of the figures:***

**Figure 1:** DSC and TG analysis of Acemetacin/(*S*)-duloxetine amorphous salt.

**Figure 2:** Powder X-Ray diffraction pattern of Acemetacin/(*S*)-duloxetine amorphous salt.

**Figure 3:** Powder X-Ray diffraction pattern of Fenbufen /(*S*)-duloxetine amorphous salt.

**Figure 4:** Powder X-Ray diffraction pattern of Ketorolac /(*S*)-duloxetine amorphous salt.

**Figure 5:** DSC and TG analysis of Nimesulide/(*S*)-duloxetine amorphous salt.

**Figure 6:** Powder X-Ray diffraction pattern of Nimesulide/(*S*)-duloxetine amorphous salt.

**Figure 7:** DSC and TG analysis of Piroxicam/(*S*)-duloxetine amorphous salt.

**Figure 8:** Powder X-Ray diffraction pattern of Piroxicam/(*S*)-duloxetine amorphous salt.

**Figure 9:** Powder X-Ray diffraction pattern of Sulindac/(*S*)-duloxetine amorphous salt.

**Figure 10:** DSC and TG analysis of diisopropyl ether hemisolvate salt of diclofenac/(*S*)-duloxetine.

**Figure 11:** Powder X-Ray diffraction pattern of diisopropyl ether hemisolvate salt of diclofenac/(*S*)-duloxetine.

**Figure 12:** DSC and TG analysis of isopropanol hemisolvate salt of diclofenac/(*S*)-duloxetine.

**Figure 13:** Powder X-Ray diffraction pattern of isopropanol hemisolvate salt of diclofenac/(*S*)-duloxetine.

**Figure 14:** DSC and TG analysis of toluene hemisolvate salt of diclofenac/(*S*)-duloxetine.

**Figure 15:** Powder X-Ray diffraction pattern of toluene hemisolvate salt of diclofenac/(*S*)-duloxetine.

**Figure 16:** DSC and TG analysis of acetone hemisolvate salt of diclofenac/(*S*)-duloxetine.

**Figure 17:** Powder X-Ray diffraction pattern of acetone hemisolvate salt of diclofenac/(*S*)-duloxetine.

**Figure 18:** DSC and TG analysis of methyl ethyl ketone hemisolvate salt of diclofenac/(*S*)-duloxetine.

**Figure 19:** Powder X-Ray diffraction pattern of methyl ethyl ketone hemisolvate salt of diclofenac/(*S*)-duloxetine.

**Figure 20:** Single Crystal XRD analysis of a single crystal of ethanol solvate of diclofenac/(*S*)-duloxetine salt.

**Figure 21:** DSC and TG analysis of non-solvated diclofenac / (*S*)-duloxetine salt.

**Figure 22:** Powder X-Ray diffraction pattern of non-solvated diclofenac / (*S*)-duloxetine salt.

## EXAMPLES

### Example 1: Acemetacin/(*S*)-duloxetine amorphous salt.

A solution of acemetacin (23.3 mg, 0.056 mmol, 1 eq) in 0.56 mL of tetrahydrofuran was added to a solution of (*S*)-duloxetine (16.7 mg, 0.056 mmol, 1 eq) in 0.36 mL of methanol. The mixture was evaporated to dryness, and the residue obtained was suspended in diisopropyl ether (2.8 mL) for 12 h at 50˚C. The oil obtained was separated from the solution and dried under vacuum for 4 h at 40˚C to give a pale yellow solid corresponding to the salt acemetacin/(*S*)-duloxetine.

The acemetacin/(*S*)-duloxetine salt has been obtained from a hot saturated solution starting with a ratio of acemetacin/(*S*)-duloxetine of 1:1. The salt has also been obtained starting with a ratio of acemetacin/(*S*)-duloxetine of 2:1 and 1:2 in diisopropyl ether.

### Characterization of acemetacin / (*S*)-duloxetine amorphous salt

**$^1$H NMR** (400 MHz, *d4*-methanol) δ: 2.29 (s, 3H), 2.39-2.48 (m, 1H), 2.56-2.66 (m, 1H), 2.71 (s, 3H), 3.16-3.23 (m, 1H), 3.27-3.34 (m, 1H), 3.80 (s, 3H), 3.85 (s, 2H), 4.49 (s, 2H), 5.94 (dd, *J* = 8 Hz, *J* = 5 Hz, 1H), 6.66 (dd, *J* = 9 Hz, *J* = 3 Hz, 1H), 6.92-6.96 (m, 3H), 7.07 (d, *J* = 3 Hz, 1H), 7.15 (d, *J* = 4 Hz, 1H), 7.26 (t, *J* = 8 Hz, 1H), 7.32 (d, *J* = 5 Hz, 1H), 7.40 (d, *J* = 8 Hz, 1H), 7.44-7.49 (m, 2H), 7.52 (d, *J* = 9 Hz, 2H), 7.65 (d, *J* = 9 Hz, 2H), 7.75-7.80 (m, 1H), 8.27-8.31 (m, 1H).
**DSC** (10˚C/min): No endothermic peak was observed. (see figure 1)
**TG** (10˚C/min): A weight loss of 0.7% presumably corresponding to water and a weight loss of 1.7% corresponding to diisopropyl ether, as detected in the $^1$H-NMR. (see figure 1)
**XRPD** (see figure 2)

### Example 2: Fenbufen/(*S*)-duloxetine amorphous salt

A solution of fenbufen (28.4 mg, 0.112 mmol, 2 eq) in 1.12 mL of tetrahydrofuran was added to a solution of (*S*)-duloxetine (16.7 mg, 0.056 mmol, 1 eq) in 0.36 mL of methanol. The mixture was evaporated to dryness, and the residue obtained was dissolved in 3.3 mL of ethanol/water (1:1) at 60˚C. The resulting solution was cooled to room

temperature. After five days, a solid was separated corresponding to fenbufen. The mother liquid was cooled to -21˚C and after three weeks a solid was obtained, which was dried under vacuum at 40˚C for 4 h to give a white solid corresponding to the fenbufen/(*S*)-duloxetine salt.

**Characterization of fenbufen/(*S*)-duloxetine amorphous salt**

**$^1$H NMR** (400 MHz, *d4*-methanol) δ: 2.39-2.50 (m, 1H), 2.57-2.66 (m, 1H), 2.62 (t, *J* = 7 Hz, 2H), 2.69 (s, 3H), 3.13-3.21 (m, 1H), 3.23-3.31 (m, 1H), 5.94 (dd, *J* = 8 Hz, *J* = 5 Hz, 1H), 6.93-6.96 (m, 2H), 7.16 (d, *J* = 3 Hz, 1H), 7.26 (t, *J* = 8 Hz, 1H), 7.31 (d, *J* = 5 Hz, 1H), 7.36-7.41 (m, 1H), 7.44-7.47 (m, 2H), 7.66 (d, *J* = 7 Hz, 2H), 7.70 (d, *J* = 7 Hz, 2H), 7.76-7.78 (m, 1H), 8.05 (d, *J* = 8 Hz, 1H), 8.28-8.31 (m, 1H).
**XRPD** (see figure 3)

**Example 3: Ketorolac/(*S*)-duloxetine amorphous salt.**

A solution of ketorolac (14.3 mg, 0.056 mmol, 1 eq) in 0.56 mL of methanol was added to a solution of (*S*)-duloxetine (16.7 mg, 0.056 mmol, 1 eq) in 0.36 mL of methanol. The mixture was evaporated to dryness, and the residue obtained was suspended in diisopropyl ether (2.3 mL) for 12 h at 50˚C. The resulting oil was separated from the solution and dried under vacuum for 4 h at 40˚C to give a brown solid corresponding to the salt ketorolac/(*S*)-duloxetine. Additionally, the salt has also been obtained starting with a ratio of ketorolac/(*S*)-duloxetine of 2:1 and 1:2.

**Characterization of ketorolac/(*S*)-duloxetine amorphous salt**

**$^1$H NMR** (400 MHz, *d4*-methanol) δ: 2.38-2.47 (m, 1H), 2.56-2.65 (m, 1H), 2.70 (s, 3H), 2.68-2.77 (m, 1H), 2.80-2.89 (m, 1H), 3.12-3.21 (m, 1H), 3.24-3.30 (m, 1H), 3.91 (dd, *J* = 8 Hz, *J* = 5 Hz, 1H), 4.29-4.36 (m, 1H), 4.49-4.56 (m, 1H), 5.95 (dd, *J* = 8 Hz, *J* = 5 Hz, 1H), 6.10 (d, *J* = 4 Hz, 1H), 6.78 (dd, *J* = 4 Hz, *J* = 2 Hz, 1H), 6.94-6.98 (m, 2H), 7.16 (d, *J* = 4 Hz, 1H), 7.27 (t, *J* = 8 Hz, 1H), 7.33 (dd, *J* = 7 Hz, *J* = 1 Hz, 1H), 7.40-7.56 (m, 7H), 7.73 (d, *J* = 7 Hz, 1H), 7.76-7.81 (m, 1H), 8.27-8.31 (m, 1H).
**XRPD** (see figure 4)

**Example 4: Nimesulide/(*S*)-duloxetine amorphous salt.**

A solution of nimesulide (17.3 mg, 0.056 mmol, 1 eq) in 0.70 mL of methanol/tetrahydrofuran (8:2) was added to a solution of (*S*)-duloxetine (16.7 mg, 0.056 mmol, 1 eq) in 0.36 mL of methanol. The mixture was evaporated to dryness, and the residue obtained was dissolved in 1.3 mL of ethanol/water (1:1) at 60˚C. The resulting solution was cooled to -21˚C. After three weeks a dense oil was separated and dried under vacuum for 4 h at 40˚C to give a yellow solid corresponding to the nimesulide/(*S*)-duloxetine salt.
The salt has also been obtained starting with a ratio of nimesulide/(*S*)-duloxetine of 1:1 and 1:2 in diisopropyl ether.

**Characterization of nimesulide / (*S*)-duloxetine amorphous salt**

**$^1$H NMR** (400 MHz, *d4*-methanol) δ: 2.31-2.40 (m, 1H), 2.50-2.59 (m, 1H), 2.55 (s, 3H), 2.97 (s, 3H), 2.98-3.13 (m, 2H), 5.90 (dd, *J* = 8 Hz, *J* = 5 Hz, 1H), 6.91-6.96 (m, 2H), 7.00-7.05 (m, 2H), 7.14-7.18 (m, 2H), 7.25 (t, *J* = 8 Hz, 1H), 7.31 (dd, *J* = 5 Hz, *J* = 2 Hz, 1H), 7.34-7.40 (m, 3H), 7.44-7.49 (m, 2H), 7.52 (d, *J* = 3 Hz, 1H), 7.64 (d, *J* = 9 Hz, 1H), 7.75-7.79 (m, 1H), 7.92 (dd, *J* = 9 Hz, *J* = 3 Hz, 1H), 8.25-8.29 (m, 1H).
**DSC** (10˚C/min): A weak endothermic peak with an onset at 58˚C was observed (see figure 5)
**TG** (10˚C/min): A weight loss of 0.5%, most probably corresponding to ethanol, was observed (see figure 5).
**XRPD** (see figure 6)

**Example 5: Piroxicam/(*S*)-duloxetine amorphous salt**

A solution of piroxicam (18.4 mg, 0.056 mmol, 1 eq) in 0.98 mL of dichloromethane was added to a solution of (*S*)-duloxetine (16.7 mg, 0.056 mmol, 1 eq) in 0.36 mL of methanol. The mixture was evaporated to dryness, and the residue obtained was dissolved in 0.8 mL of ethanol/water (1:1) at 60˚C. The resulting solution was cooled to -21˚C. After four weeks a solid was separated and dried under vacuum for 4 h at 40˚C to give a yellow solid corresponding to the piroxicam/(*S*)-duloxetine salt.
The piroxicam/(*S*)-duloxetine salt has also been obtained from a hot saturated solution starting with a ratio of piroxicam/(*S*)-duloxetine of 1:1 in ethanol/water (1:1) and toluene, and with a ratio of piroxicam/(*S*)-duloxetine of 2:1 in

ethanol/water (1:1).

**Characterization of piroxicam/(*S*)-duloxetine amorphous salt**

**$^1$H NMR** (400 MHz, *d4*-methanol) δ: 2.39-2.48 (m, 1H), 2.56-2.65 (m, 1H), 2.72 (s, 3H), 2.86 (s, 3H), 3.17-3.24 (m, 1H), 3.28-3.35 (m, 1H), 5.94 (dd, *J* = 8 Hz, *J* = 5 Hz, 1H), 6.92-6.99 (m, 3H), 7.15 (d, *J* = 3 Hz, 1H), 7.26 (t, *J* = 8 Hz, 1H), 7.31 (d, *J* = 6 Hz, 1H), 7.40 (d, *J* = 8 Hz, 1H), 7.44-7.49 (m, 2H), 7.59-7.80 (m, 5H), 8.0 (d, *J* = 8 Hz, 1H), 8.19 (d, *J* = 5 Hz, 1H), 8.25-8.29 (m, 2H).
DSC (10˚C/min): A broad endothermic peak with an onset at 62˚C was observed (see figure 7).
**TG** (10˚C/min): A weight loss of 1.4% corresponding to ethanol, as detected in the $^1$H-NMR (see figure 7).
**XRPD** (see figure 8).

**<u>Example 6: Sulindac/(*S*)-duloxetine amorphous salt</u>**

A solution of sulindac (40.0 mg, 0.112 mmol, 2 eq) in 1.12 mL of tetrahydrofuran was added to a solution of (*S*)-duloxetine (16.7 mg, 0.056 mmol, 1 eq) in 0.36 mL of methanol. The mixture was evaporated to dryness, and the residue obtained was dissolved in 3.3 mL of ethanol/water (1:1) at 60˚C. The resulting solution was cooled to room temperature, and after five days, a solid was formed corresponding to sulindac. The mother liquid was cooled to -21 ˚C for three weeks, and the resulting solid was dried under vacuum at 40˚C for 4 h to give a yellow-orange solid corresponding to the sulindac/(*S*)-duloxetine salt.
The salt has also been obtained starting with a ratio of piroxicam/(*S*)-duloxetine of 2:1, 1:1 and 1:2 in diisopropyl ether.

**Characterization of sulindac/(*S*)-duloxetine amorphous salt**

**$^1$H NMR** (400 MHz, *d4*-methanol) δ: 2.19 (s, 3H), 2.38-2.46 (m, 1H), 2.55-2.65 (m, 1H), 2.70 (s, 3H), 2.87 (s, 3H), 3.15-3.22 (m, 1H), 3.25-3.32 (m, 1H), 3.49 (s, 2H), 5.93 (dd, *J* = 8 Hz, *J* = 5 Hz, 1H), 6.51 (td, *J* = 9 Hz, *J* = 1 Hz, 1H), 6.93-6.99 (m, 2H), 7.10 (dd, *J* = 8 Hz, *J* = 4 Hz, 1H), 7.15 (d, *J* = 5 Hz, 1H), 7.23 (s, 1H), 7.27 (t, *J* = 8 Hz, 1H), 7.32 (d, *J* = 4 Hz, 1H), 7.41 (d, *J* = 8 Hz, 1H), 7.45-7.49 (m, 2H), 7.71 (d, *J* = 8 Hz, 2H), 7.77 (d, *J* = 8 Hz, 2H), 7.78-7.80 (m, 1H), 8.26-8.30 (m, 1H).
**XRPD** (see figure 9).

**<u>Example 7: Diisopropyl ether hemisolvate salt of diclofenac/(*S*)-duloxetine.</u>**

A solution of (*S*)-duloxetine (1.23 g, 4.14 mmol, 1 eq) in 7 mL of diisopropyl ether was added to a suspension of diclofenac (1.88 g, 4.01 mmol, 1 eq) in 8 mL of diisopropyl ether at 68˚C. The resulting mixture was stirred 0.5 h at 68˚C, 1 h at 50˚C and, finally, 1 h at room temperature. The resulting solid was filtered and dried under vacuum at 40˚C for 4 h to give an abundant white solid (2.22 g, 86% yield) corresponding to the diisopropyl ether hemisolvate salt diclofenac/(*S*)-duloxetine (phase 1).
The diisopropyl ether hemisolvate salt of diclofenac/(*S*)-duloxetine has also been obtained by crystallization from a hot saturated solution starting with diclofenac-Na and (*S*)-duloxetine.HCl with a ratio of diclofenac-Na/(*S*)-duloxetine·HCl of 2:1, 1:1 and 1:2 in diisopropyl ether.

**Characterization of the diisopropyl ether hemisolvate salt of diclofenac/(*S*)-duloxetine**

**$^1$H NMR** (400 MHz, *d4*-methanol) δ: 1.12 (d, *J* = 6 Hz, 6H), 2.37-2.46 (m, 1H), 2.55-2.64 (m, 1H), 2.67 (s, 3H), 3.12-3.19 (m, 1H), 3.22-3.27 (m, 1H), 3.63 (s, 2H), 3.70 (hept, *J* = 6 Hz, 1H), 5.93 (dd, *J* = 8 Hz, *J* = 5 Hz, 1H), 6.36 (d, *J* = 8 Hz, 1H), 6.82 (t, *J* = 8 Hz, 1H), 6.93-6.98 (m, 3H), 7.02 (t, *J* = 8 Hz, 1H), 7.15 (d, *J* = 3 Hz, 1H), 7.18 (d, *J* = 8 Hz, 1H), 7.27 (t, *J* = 8 Hz, 1H), 7.32 (m, 1H), 7.35 (d, *J* = 8 Hz, 2H), 7.41 (d, *J* = 8 Hz, 1H), 7.45-7.49 (m, 2H), 7.76-7.78 (m, 1H), 8.27-8.30 (m, 1H).
**DSC** (10˚C/min): A broad endothermic peak with an onset at 83˚C was observed (see figure 10).
**TG** (10˚C/min): A weight loss of 5.6% at 75˚C corresponding to diisopropyl ether, as detected in the $^1$H-NMR (see figure 10).
**XRPD**

**Table 1.** List of selected peaks obtained by powder X-ray diffraction of diclofenac / (*S*)-duloxetine diisopropyl ether hemisolvate salt (see figure 11).

| Angle 2θ[1] (°) | d value (Å) | Relative Intensity % | | Angle 2θ[1] (°) | d value (Å) | Relative Intensity % |
|---|---|---|---|---|---|---|
| 5.5 | 15.93 | 50.1 | | 15.2 | 5.82 | 11.7 |
| 8.6 | 10.30 | 18.1 | | 15.5 | 5.71 | 10.3 |
| 8.9 | 9.91 | 16.0 | | 16.5 | 5.38 | 10.4 |
| 9.4 | 9.36 | 13.3 | | 17.0 | 5.20 | 32.8 |
| 10.1 | 8.71 | 14.8 | | 17.3 | 5.13 | 27.1 |
| 11.0 | 8.04 | 22.0 | | 17.5 | 5.07 | 21.7 |
| 11.2 | 7.92 | 25.9 | | 17.6 | 5.03 | 48.1 |
| 11.9 | 7.41 | 67.4 | | 18.5 | 4.79 | 73.9 |
| 12.1 | 7.32 | 14.2 | | 19.1 | 4.64 | 86.0 |
| 12.3 | 7.17 | 24.8 | | 19.3 | 4.60 | 67.4 |
| 12.7 | 6.95 | 79.8 | | 19.5 | 4.54 | 30.7 |
| 13.3 | 6.67 | 13.0 | | 19.7 | 4.50 | 33.4 |
| 13.9 | 6.36 | 27.6 | | 19.9 | 4.46 | 100.0 |
| 14.1 | 6.29 | 11.2 | | 20.2 | 4.40 | 14.1 |
| 14.7 | 6.04 | 16.2 | | 20.3 | 4.36 | 34.3 |
| [1]The 2θ values were obtained using copper radiation ($Cu_{K\alpha 1}$ 1.54060Å) | | | | | | |

**Example 8: Isopropanol hemisolvate salt of diclofenac/(*S*)-duloxetine.**

A solution of (*S*)-duloxetine·HCl (15.7 mg, 0.047 mmol, 1 eq) in 0.55 mL of methanol was added to a solution of diclofenac-Na (35.0 mg, 0.094 mmol, 2 eq) in 1,1 mL of methanol. The mixture was evaporated to dryness, and the residue obtained was suspended in isopropanol (2.5 mL) for 0.5 h at 75°C. The solid obtained (NaCl) was separated from the solution and the mother liquid was evaporated at room temperature. The resulting white solid corresponded to the isopropanol hemisolvate salt of diclofenac/(*S*)-duloxetine (phase 2).

The isopropanol hemisolvate salt of diclofenac/(*S*)-duloxetine has also been obtained by crystallization from a hot saturated solution starting with diclofenac and (*S*)-duloxetine with a ratio of diclofenac/(*S*)-duloxetine of 1:1 in isopropanol.

**Characterization of the isopropanol hemisolvate salt of diclofenac/(*S*)-duloxetine**

**$^1$H NMR** (400 MHz, *d4*-methanol) δ: 1.15 (d, J = 6 Hz, 3H), 2.37-2.46 (m, 1H), 2.55-2.64 (m, 1H), 2.67 (s, 3H), 3.12-3.19 (m, 1H), 3.22-3.27 (m, 1H), 3.63 (s, 2H), 3.92 (m, 0.5H), 5.93 (dd, *J* = 8 Hz, *J* = 5 Hz, 1H), 6.36 (d, *J* = 8 Hz, 1H), 6.36 (t, *J* = 8 Hz, 1H), 6.93-6.98 (m, 2H), 7.02 (t, *J* = 8 Hz, 1H), 7.15 (d, *J* = 3 Hz, 1H), 7.18 (d, *J* = 8 Hz, 1H), 7.27 (t, *J* = 8 Hz, 1H), 7.32 (d, *J* = 6 Hz, 1H), 7.35 (d, *J* = 8 Hz, 1H), 7.41 (d, *J* = 8 Hz, 1H), 7.45-7.49 (m, 2H), 7.76-7.78 (m, 1H), 8.27-8.30 (m, 1H).

**DSC** (10°C/min): A broad endothermic peak with an onset at 73°C was observed (see figure 12).

**TG** (10°C/min): A weight loss of 4% at 85°C corresponding to isopropanol, as detected in the $^1$H-NMR (see figure 12).

**XRPD**

**Table 2.** List of selected peaks obtained by powder X-ray diffraction of diclofenac / (*S*)-duloxetine isopropanol hemisolvate salt (see figure 13).

| Angle 2θ[1] (°) | d value (Å) | Relative Intensity % | | Angle 2θ[1] (°) | d value (Å) | Relative Intensity % |
|---|---|---|---|---|---|---|
| 5.5 | 15.99 | 73 | | 19.2 | 4.61 | 46.8 |

(continued)

| Angle 2θ[1] (°) | d value (Å) | Relative Intensity % | | Angle 2θ[1] (°) | d value (Å) | Relative Intensity % |
|---|---|---|---|---|---|---|
| 9.0 | 9.84 | 52.4 | | 19.7 | 4.50 | 57.1 |
| 9,8 | 9.00 | 30 | | 19.8 | 4.48 | 62.7 |
| 11.0 | 8.05 | 35.4 | | 20.2 | 4.40 | 93.2 |
| 11.1 | 7.95 | 34.4 | | 21.4 | 4.16 | 72.5 |
| 12.1 | 7.29 | 31.1 | | 22.1 | 4.02 | 33.1 |
| 13.0 | 6.80 | 100 | | 23.6 | 3.77 | 23 |
| 13.7 | 6.47 | 41 | | 23.8 | 3.73 | 27.4 |
| 13.8 | 6.42 | 40.4 | | 24.4 | 3.64 | 50.9 |
| 16.3 | 5.44 | 29.3 | | 24.7 | 3.61 | 29 |
| 17.0 | 5.20 | 31.4 | | 25.0 | 3.56 | 39.9 |
| 17.2 | 5.14 | 44.1 | | 25.2 | 3.53 | 29.8 |
| 18.0 | 4.91 | 45.9 | | 25.4 | 3.50 | 37 |
| 18.4 | 4.82 | 41 | | 26.2 | 3.40 | 33.7 |
| 18.6 | 4.76 | 38.2 | | 26.6 | 3.34 | 22.3 |
| [1]The 2θ values were obtained using copper radiation ($Cu_{K\alpha 1}$ 1.54060Å) | | | | | | |

**Example 9: Toluene hemisolvate salt of diclofenac/(*S*)-duloxetine.**

A solution of (*S*)-duloxetine·HCl (15.7 mg, 0.047 mmol, 1 eq) in 0.55 mL of methanol was added to a solution of diclofenac-Na (35.0 mg, 0.094 mmol, 2 eq) in 1,1 mL of methanol. The mixture was evaporated to dryness, and the residue obtained was suspended in toluene (3 mL) for 0.5 h at 75°C. The solid obtained (NaCl) was separated from the solution and the mother liquid was evaporated at room temperature. The resulting white solid corresponded to the toluene hemisolvate salt of diclofenac/(*S*)-duloxetine (phase 3).

The toluene hemisolvate salt of diclofenac/(*S*)-duloxetine has also been obtained by crystallization from a hot saturated solution starting with diclofenac and (*S*)-duloxetine with a ratio of diclofenac/(*S*)-duloxetine of 1:1 in toluene.

**Characterization of the toluene hemisolvate salt of diclofenac/(*S*)-duloxetine.**

**$^1$H NMR** (400 MHz, *d4*-methanol) δ: 2.32 (s, 1.5H) 2.37-2.46 (m, 1H), 2.55-2.64 (m, 1H), 2.67 (s, 3H), 3.12-3.19 (m, 1H), 3.22-3.27 (m, 1H), 3.63 (s, 2H), 5.93 (dd, *J* = 8 Hz, *J* = 5 Hz, 1H), 6.36 (d, *J* = 8 Hz, 1H), 6.36 (t, *J* = 8 Hz, 1H), 6.94-7.05 (m, 4H),7.08-7.50 (m, 11.5H), 7.76-7.78 (m, 1H), 8.27-8.30 (m, 1H).
**DSC** (10°C/min): A broad endothermic peak with an onset at 78°C was observed (see figure 14).
**TG** (10°C/min): A weight loss of 6.2% at 74°C corresponding to toluene, as detected in the $^1$H-NMR (see figure 14).
**XRPD**

**Table 3.** List of selected peaks obtained by powder X-ray diffraction of diclofenac / (*S*)-duloxetine toluene hemisolvate salt (see figure 15).

| Angle 2θ[1] (°) | d value (Å) | Relative Intensity % | | Angle 2θ[1] (°) | d value (Å) | Relative Intensity % |
|---|---|---|---|---|---|---|
| 5.2 | 17.02 | 35.8 | | 19.1 | 4.63 | 27.2 |
| 9.0 | 9.78 | 17.4 | | 19.7 | 4.50 | 38.3 |
| 9.8 | 9.06 | 15.6 | | 20.0 | 4.44 | 100 |
| 10.4 | 8.48 | 11.5 | | 20.1 | 4.41 | 32.4 |
| 12.3 | 7.21 | 29.9 | | 20.7 | 4.28 | 28.4 |

(continued)

| Angle 2θ[1] (˚) | d value (Å) | Relative Intensity % | | Angle 2θ[1] (˚) | d value (Å) | Relative Intensity % |
|---|---|---|---|---|---|---|
| 12.6 | 7.03 | 41 | | 21.0 | 4.23 | 10.9 |
| 13.3 | 6.64 | 20.3 | | 21.4 | 4.15 | 18.8 |
| 14.0 | 6.30 | 41.3 | | 21.5 | 4.13 | 29.9 |
| 14.9 | 5.95 | 10.7 | | 22.6 | 3.94 | 36.4 |
| 16.5 | 5.38 | 10.9 | | 22.9 | 3.88 | 9.4 |
| 17.4 | 5.10 | 12.4 | | 23.1 | 3.85 | 11.4 |
| 17.8 | 4.99 | 20.8 | | 23.5 | 3.79 | 9.9 |
| 18.2 | 4.88 | 40.7 | | 24.3 | 3.66 | 21.8 |
| 18.5 | 4.80 | 26.6 | | 24.5 | 3.63 | 50.3 |
| 18.9 | 4.69 | 59.1 | | 25.4 | 3.51 | 38.5 |
| [1]The 2θ values were obtained using copper radiation ($Cu_{K\alpha 1}$ 1.54060Å) | | | | | | |

**Example 10: Acetone hemisolvate salt of diclofenac/(*S*)-duloxetine.**

A solution of (*S*)-duloxetine (20 mg, 0.067 mmol, 1 eq) in 0.55 mL of methanol was added to a solution of diclofenac (19.9 mg, 0.067 mmol, 1 eq) in 1.1 mL of methanol. The mixture was evaporated to dryness, and the residue obtained was dissolved in acetone (0.3 mL) at 55˚C. The solution was evaporated at -18˚C. The resulting white solid corresponded to the acetone hemisolvate salt of diclofenac/(*S*)-duloxetine (phase 4).

**Characterization of the acetone hemisolvate salt of diclofenac/(*S*)-duloxetine.**

**$^1$H NMR** (400 MHz, *d4*-methanol) δ: 2.15 (s, 3H), 2.37-2.46 (m, 1H), 2.55-2.64 (m, 1H), 2.67 (s, 3H), 3.12-3.19 (m, 1H), 3.22-3.27 (m, 1H), 3.63 (s, 2H), 5.93 (dd, *J* = 8 Hz, *J* = 5 Hz, 1H), 6.36 (d, *J* = 8 Hz, 1H), 6.36 (t, *J* = 8 Hz, 1H), 6.93-6.98 (m, 2H), 7.02 (t, *J* = 8 Hz, 1H), 7.15 (d, *J* = 3 Hz, 1H), 7.18 (d, *J* = 8 Hz, 1H), 7.27 (t, *J* = 8 Hz, 1H), 7.32 (d, *J* = 6 Hz, 1H), 7.35 (d, *J* = 8 Hz, 1H), 7.41 (d, *J* = 8 Hz, 1H), 7.45-7.49 (m, 2H), 7.76-7.78 (m, 1H), 8.27-8.30 (m, 1H).
**DSC** (10˚C/min): An endothermic peak with an onset at 101˚C was observed (see figure 16).
**TG** (10˚C/min): A weight loss of 5.6% at 100˚C corresponding to acetone, as detected in the $^1$H-NMR (see figure 16).
**XRPD**

**Table 4.** List of selected peaks obtained by powder X-ray diffraction of diclofenac / (*S*)-duloxetine acetone hemisolvate salt (see figure 17).

| Angle 2θ[1] (˚) | d value (Å) | Relative Intensity % | | Angle 2θ[1] (˚) | d value (Å) | Relative Intensity % |
|---|---|---|---|---|---|---|
| 5.5 | 16.09 | 56.2 | | 19.2 | 4.62 | 36.2 |
| 9.0 | 9.84 | 25.3 | | 19.4 | 4.56 | 17.7 |
| 9.8 | 9.00 | 21.2 | | 19.8 | 4.49 | 99.8 |
| 11.0 | 8.01 | 52.7 | | 19.8 | 4.47 | 84.3 |
| 12.1 | 7.29 | 14.2 | | 20.1 | 4.42 | 94.2 |
| 12.9 | 6.83 | 83.8 | | 21.3 | 4.17 | 100 |
| 13.7 | 6.44 | 82.9 | | 21.8 | 4.07 | 16.6 |
| 15.8 | 5.60 | 11.5 | | 22.2 | 4.00 | 61.7 |
| 16.3 | 5.43 | 13.2 | | 23.7 | 3.75 | 45.6 |
| 17.1 | 5.19 | 28.3 | | 24.4 | 3.64 | 48.4 |

(continued)

| Angle 2θ[1] (°) | d value (Å) | Relative Intensity % | | Angle 2θ[1] (°) | d value (Å) | Relative Intensity % |
|---|---|---|---|---|---|---|
| 17.2 | 5.14 | 48.3 | | 24.7 | 3.60 | 36.1 |
| 18.1 | 4.91 | 40.7 | | 25.2 | 3.53 | 22.5 |
| 18.3 | 4.84 | 30.7 | | 25.5 | 3.49 | 38.7 |
| 18.5 | 4.78 | 37.2 | | 26.1 | 3.41 | 15.2 |
| 18.7 | 4.75 | 19.7 | | 26.2 | 3.39 | 18.5 |
| [1]The 2θ values were obtained using copper radiation ($Cu_{K\alpha1}$ 1.54060Å) | | | | | | |

**Example 11: Methyl ethyl ketone hemisolvate salt of diclofenac/(*S*)-duloxetine.**

A solution of (*S*)-duloxetine (20 mg, 0.067 mmol, 1 eq) in 0.55 mL of methanol was added to a solution of diclofenac (19.9 mg, 0.067 mmol, 1 eq) in 1.1 mL of methanol. The mixture was evaporated to dryness, and the residue obtained was dissolved in methyl ethyl ketone (0.3 mL) at 55°C. The solution was cooled to -18°C, and after four days a white solid precipitated. The resulting white solid corresponded to the methyl ethyl ketone hemisolvate salt of diclofenac/(*S*)-duloxetine (phase 6).

**Characterization of the methyl ethyl ketone hemisolvate salt of diclofenac/(*S*)-duloxetine.**

**$^1$H NMR** (400 MHz, *d4*-methanol) δ: 1.01 (t, J = 7.4 Hz, 1.5H), 2.13 (s, 1.5H), 2.37-2.64 (m, 3H), 2.67 (s, 3H), 3.12-3.19 (m, 1H), 3.22-3.27 (m, 1H), 3.63 (s, 2H), 5.93 (dd, *J* = 8 Hz, *J* = 5 Hz, 1H), 6.36 (d, *J* = 8 Hz, 1H), 6.36 (t, *J* = 8 Hz, 1H), 6.93-6.98 (m, 2H), 7.02 (t, *J* = 8 Hz, 1H), 7.15 (d, *J* = 3 Hz, 1H), 7.18 (d, *J* = 8 Hz, 1H), 7.27 (t, *J* = 8 Hz, 1H), 7.32 (d, *J* = 6 Hz, 1H), 7.35 (d, *J* = 8 Hz, 1H), 7.41 (d, *J* = 8 Hz, 1H), 7.45-7.49 (m, 2H), 7.76-7.78 (m, 1H), 8.27-8.30 (m, 1H).
**DSC** (10°C/min): A broad endothermic peak with an onset at 80°C was observed (see figure 18).
**TG** (10°C/min): A weight loss of 6% at 77°C corresponding to methyl ethyl ketone, as detected in the $^1$H-NMR (see figure 18).
**XRPD**

**Table 5.** List of selected peaks obtained by powder X-ray diffraction of diclofenac / (*S*)-duloxetine methyl ethyl ketone hemisolvate salt (see figure 19).

| Angle 2θ[1] (°) | d value (Å) | Relative Intensity % | | Angle 2θ[1] (°) | d value (Å) | Relative Intensity % |
|---|---|---|---|---|---|---|
| 5.3 | 16.70 | 59 | | 18.8 | 4.71 | 43.8 |
| 5.8 | 15.17 | 36.2 | | 19.1 | 4.64 | 27.7 |
| 9.1 | 9.67 | 29.4 | | 19.4 | 4.58 | 21.1 |
| 9.6 | 9.16 | 19.9 | | 19.9 | 4.45 | 54.4 |
| 10.6 | 8.31 | 20.7 | | 20.1 | 4.40 | 100 |
| 12.3 | 7.20 | 37.3 | | 20.6 | 4.30 | 25.6 |
| 12.6 | 7.02 | 37.2 | | 20.9 | 4.25 | 25.5 |
| 13.4 | 6.60 | 27.5 | | 21.5 | 4.12 | 25.1 |
| 13.7 | 6.44 | 25.7 | | 21.9 | 4.05 | 43.1 |
| 14.0 | 6.30 | 33.2 | | 22.4 | 3.96 | 22.3 |
| 16.4 | 5.41 | 25.6 | | 22.8 | 3.90 | 20.7 |
| 16.8 | 5.27 | 18.9 | | 24.6 | 3.62 | 46.9 |
| 17.3 | 5.11 | 29.5 | | 25.4 | 3.51 | 29.4 |
| 18.1 | 4.90 | 37.2 | | 25.6 | 3.48 | 24.8 |

(continued)

| Angle 2θ[1] (°) | d value (Å) | Relative Intensity % | | Angle 2θ[1] (°) | d value (Å) | Relative Intensity % |
|---|---|---|---|---|---|---|
| 18.4 | 4.83 | 29.3 | | 26.4 | 3.37 | 30.9 |
| [1]The 2θ values were obtained using copper radiation ($Cu_{K\alpha 1}$ 1.54060Å) | | | | | | |

**Example 12: Non-solvated diclofenac/(*S*)-duloxetine salt.**

The diisopropyl hemisolvate salt of diclofenac/(*S*)-duloxetine (54.3 mg) was grinded with two drops of ethanol for 10 min. The resulting solid corresponded to the ethanol solvate of diclofenac/(*S*)-duloxetine salt. The structure of the ethanol solvate of diclofenac/(*S*)-duloxetine salt has been determined by SCXRD (see figure 20).

**Single Crystal XRD analysis of a single crystal of ethanol solvate of diclofenac/(*S*)-duloxetine salt (see figure 20):**

The crystal structure was determined from single crystal X-ray diffraction data. The measured crystal was selected using a Zeiss stereomicroscope using polarized light and prepared under inert conditions immersed in perfluoropolyether as protecting oil for manipulation. Crystal structure determination was carried out using a Bruker-Nonius diffractometer equipped with a APPEX 2 4K CCD area detector, a FR591 rotating anode with $Mo_{K\alpha}$ radiation, Montel mirrors as monochromator and a Kryoflex low temperature device (T = 100 K). Fullsphere data collection omega and phi scans. Programs used: Data collection Apex2 V. 1.0-22 (Bruker-Nonius 2004), data reduction Saint + Version 6.22 (Bruker-Nonius 2001) and absorption correction SADABS V. 2.10 (2003). Crystal structure solution was achieved using direct methods as implemented in SHELXTL Version 6.10 (Sheldrick, Universtität Göttingen (Germany), 2000) and visualized using XP program. Missing atoms were subsequently located from difference Fourier synthesis and added to the atom list. Least-squares refinement on $F_0^2$ using all measured intensities was carried out using the program SHELXTL Version 6.10 (Sheldrick, Universtität Göttingen (Germany), 2000). All non hydrogen atoms were refined including anisotropic displacement parameters. In Figure 20 the structure of the salt is shown. Crystal data and structure refinement for ethanol solvate of diclofenac/(*S*)-duloxetine salt is given in the following table 6.

**Table 6:** Most relevant structural data of the SCXRD analysis of ethanol solvate diclofenac/(*S*)-duloxetine salt.

| | |
|---|---|
| Crystal system | Monoclinic |
| Space group: | $P2_1$ |
| a (A) | 9.4952(5) |
| b (A) | 10.6992(5) |
| c (Å) | 15.7550(9) |
| α (°) | 90.00 |
| β (°) | 96.490(2) |
| γ (°) | 90.00 |
| z | 2 |
| Volume (Å$^3$) | 1593.22(14) |

After drying the ethanol solvate diclofenac/(*S*)-duloxetine salt under vacuum at 40°C for 4 h, a new phase was obtained that was characterized as the non-solvated diclofenac/(*S*)-duloxetine salt.

**Characterization of non-solvated diclofenac/(*S*)-duloxetine anhydrous salt**

**$^1$H NMR** (400 MHz, *d4*-methanol) δ: 2.37-2.46 (m, 1H), 2.55-2.64 (m, 1H), 2.67 (s, 3H), 3.12-3.19 (m, 1H), 3.22-3.27 (m, 1H), 3.63 (s, 2H), 5.93 (dd, *J* = 8 Hz, *J* = 5 Hz, 1H), 6.36 (d, *J* = 8 Hz, 1H), 6.82 (t, *J* = 8 Hz, 1H), 6.93-6.98 (m, 3H), 7.02 (t, *J*= 8 Hz, 1H), 7.15 (d, *J*= 3 Hz, 1H), 7.18 (d, *J* = 8 Hz, 1H), 7.27 (t, *J* = 8 Hz, 1H), 7.32 (m, 1H), 7.35 (d, *J* = 8 Hz, 2H), 7.41 (d, *J* = 8 Hz, 1H), 7.45-7.49 (m, 2H), 7.76-7.78 (m, 1H), 8.27-8.30 (m, 1H).
**DSC** (10°C/min): A broad endothermic peak with an onset at 75°C was observed (see figure 21).
**TG** (10°C/min): No weight loss was observed at temperatures below 180°C, indicating the absence of crystallization

solvent (see figure 21).

**XRPD**

**Table 6.** List of selected peaks obtained by powder X-ray diffraction of non-solvated diclofenac / (*S*)-duloxetine salt (see figure 22).

| Angle 2θ[1] (°) | d value (Å) | Relative Intensity % | | Angle 2θ[1] (°) | d value (Å) | Relative Intensity % |
|---|---|---|---|---|---|---|
| 5,6 | 15,83 | 100,0 | | 20,3 | 4,37 | 56,9 |
| 9,2 | 9,59 | 54,0 | | 20,6 | 4,31 | 54,7 |
| 9,9 | 8,93 | 18,2 | | 21,6 | 4,12 | 47,7 |
| 11,2 | 7,86 | 56,4 | | 22,6 | 3,92 | 28,7 |
| 12,4 | 7,15 | 24,0 | | 24,1 | 3,70 | 22,3 |
| 12,7 | 6,95 | 18,3 | | 24,9 | 3,57 | 31,1 |
| 13,2 | 6,69 | 86,9 | | 25,4 | 3,51 | 24,7 |
| 13,9 | 6,36 | 49,5 | | 26,0 | 3,43 | 23,2 |
| 16,2 | 5,48 | 15,7 | | 26,6 | 3,34 | 15,1 |
| 16,4 | 5,40 | 16,8 | | 27,2 | 3,27 | 14,6 |
| 17,3 | 5,12 | 39,8 | | 27,7 | 3,22 | 14,9 |
| 18,4 | 4,81 | 29,0 | | 29,2 | 3,05 | 20,4 |
| 18,8 | 4,72 | 32,2 | | 32,0 | 2,80 | 10,7 |
| 19,5 | 4,55 | 21,3 | | 32,6 | 2,74 | 15,2 |
| 20,0 | 4,45 | 36,5 | | 33,7 | 2,66 | 13,5 |
| [1]The 2θ values were obtained using copper radiation ($Cu_{K\alpha 1}$ 1.54060Å) | | | | | | |

## Claims

1. A salt comprising duloxetine either as a free base or as its physiologically acceptable salt and at least one NSAID

2. A salt according to claim 1, wherein the NSAID is selected from salicylates, anthranilates, arylacetic acids/arylalkanoic acids, oxicams and arylpropionic acids, ketorolac, fenbufen and nimesulide; or their stereoisomers or solvates.

3. A salt according to claim 2, wherein the NSAID is selected from acemetacin, fenbufen, ketorolac, nimesulide, piroxicam, sulindac, and diclofenac; or their stereoisomers or solvates.

4. A salt according to any of claims 1 to 3 selected from

   - Acemetacin/(*S*)-duloxetine amorphous salt.
   - Fenbufen/(*S*)-duloxetine amorphous salt.
   - Ketorolac/(*S*)-duloxetine amorphous salt.
   - Nimesulide/(*S*)-duloxetine amorphous salt.
   - Piroxicam/(*S*)-duloxetine amorphous salt.
   - Sulindac/(*S*)-duloxetine amorphous salt.
   - Diisopropyl ether hemisolvate salt of diclofenac/(*S*)-duloxetine.
   - Isopropanol hemisolvate salt of diclofenac/(*S*)-duloxetine.
   - Toluene hemisolvate salt of diclofenac/(*S*)-duloxetine.
   - Acetone hemisolvate salt of diclofenac/(*S*)-duloxetine.
   - Methyl ethyl ketone hemisolvate salt of diclofenac/(*S*)-duloxetine.
   - Ethanol solvate of diclofenac/(*S*)-duloxetine salt.
   - Non-solvated diclofenac/(*S*)-duloxetine salt.

**5.** Diisopropyl ether hemisolvate salt of diclofenac/(*S*)-duloxetine according to claim 4, **characterized in that** it shows an X-Ray powder diffraction pattern with peaks [2θ] at 5.5, 8.6, 8.9, 9.4, 10.1, 11.0, 11.2, 11.9, 12.1, 12.3, 12.7, 13.3, 13.9, 14.1, 14.7, 15.2, 15.5, 16.5, 17.0, 17.3, 17.5, 17.6, 18.5, 19.1, 19.3, 19.5, 19.7, 19.9, 20.2 and 20.3 [°] with the 2θ values being obtained using copper radiation ($Cu_{K\alpha1}$ 1.54060Å).

**6.** Isopropanol hemisolvate salt of diclofenac/(*S*)-duloxetine according to claim 4, **characterized in that** it shows an X-Ray powder diffraction pattern with peaks [2θ] at 5.5, 9.0, 9.8, 11.0, 11.1, 12.1, 13.0, 13.7, 13.8, 16.3, 17.0, 17.2, 18.0, 18.4, 18.6, 19.2, 19.7, 19.8, 20.2, 21.4, 22.1, 23.6, 23.8, 24.4, 24.7, 25.0, 25.2, 25.4, 26.2, and 26.6 [°] with the 2θ values being obtained using copper radiation ($Cu_{K\alpha1}$ 1.54060Å).

**7.** Toluene hemisolvate salt of diclofenac/(*S*)-duloxetine according to claim 4, **characterized in that** it shows an X-Ray powder diffraction pattern with peaks [2θ] at 5.2, 9.0, 9.8, 10.4, 12.3, 12.6, 13.3, 14.0, 14.9, 16.5, 17.4, 17.8, 18.2, 18.5, 18.9, 19.1, 19.7, 20.0, 20.1, 20.7, 21.0, 21.4, 21.5, 22.6, 22.9, 23.1, 23.5, 24.3, 24.5, and 25.4 [°] with the 2θ values being obtained using copper radiation ($Cu_{K\alpha1}$ 1.54060Å).

**8.** Acetone hemisolvate salt of diclofenac/(*S*)-duloxetine according to claim 4, **characterized in that** it shows an X-Ray powder diffraction pattern with peaks [2θ] at 5.5, 9.0, 9.8, 11.0, 12.1, 12.9, 13.7, 15.8, 16.3, 17.1, 17.2, 18.1, 18.3, 18.5, 18.7, 19.2, 19.4, 19.8, 20.1, 21.3, 21.8, 22.2, 23.7, 24.4, 24.7, 25.2, 25.5, 26.1, and 26.2 [°] with the 2θ values being obtained using copper radiation ($Cu_{K\alpha1}$ 1.54060Å).

**9.** Methyl ethyl ketone hemisolvate salt of diclofenac/(*S*)-duloxetine according to claim 4, **characterized in that** it shows an X-Ray powder diffraction pattern with peaks [2θ] at 5.3, 5.8, 9.1, 9.6, 10.6, 12.3, 12.6, 13.4, 13.7, 14.0, 16.4, 16.8, 17.3, 18.1, 18.4, 18.8, 19.1, 19.4, 19.9, 20.1, 20.6, 20.9, 21.5, 21.9, 22.4, 22.8, 24.6, 25.4, 25.6, and 26,4 [°] with the 2θ values being obtained using copper radiation ($Cu_{K\alpha1}$ 1.54060Å).

**10.** Ethanol solvate of diclofenac / (S)-duloxetine salt according to claim 4, **characterized in that** its single crystal has a monoclinic unit cell with the following dimensions:

$$a = 9.4952(5)\ Å$$

$$b = 10.6992(5)\ Å$$

$$c = 15.7550(9)\ Å$$

$$\alpha = 90.00°$$

$$\beta = 96.490(2)°$$

$$\gamma = 90.00°.$$

**11.** Non-solvated diclofenac/(*S*)-duloxetine salt according to claim 4, **characterized in that** it shows an X-Ray powder diffraction pattern with peaks [2θ] at 5.6, 9.2, 9.9, 11.2, 12.4, 12.7, 13.2, 13.9, 16.2, 16.4, 17.3, 18.4, 18.8, 19.5, 20.0, 20.3, 20.6, 21.6, 22.6, 24.1, 24.9, 25.4, 26.0, 26.6, 27.2, 27.7, 29.2, 32.0, 32.6 and 33.7 [°] with the 2θ values being obtained using copper radiation ($Cu_{K\alpha1}$ 1.54060Å).

**12.** Process for the production of a salt according to claim 1 comprising the steps of:

a) dissolving or suspending the NSAID in organic solvent 1;

b) either before or after step (a) dissolving duloxetine either as a free base or as a salt in organic solvent 2,
c) mixing the solutions from step (a) and (b);
d) evaporating the solvent(s) of the mixture from step (c) to dryness,
e) optionally dissolving the residue of step (d) in organic solvent 3:
f) optionally removing the organic solvent 3;

**13.** Process according to claim 11, wherein

• the organic solvent 1 is selected from tetrahydrofuran or methanol or mixtures thereof; or from dichloromethane, and diisopropyl ether; and/or
• the organic solvent 2 is selected from methanol, and diisopropyl ether; and/or
• the organic solvent 3 is selected from diisopropyl ether, ethanol/water (1:1), or isopropanol, toluene, acetone, and methyl ethyl ketone.

**14.** Pharmaceutical composition **characterized in that** it comprises a therapeutically effective amount of the salt according to any one of claims 1 to 10 in a physiologically acceptable medium.

**15.** Use of a salt according to any one of claims 1 to 10 in the manufacture of a medicament for the treatment of pain, preferably acute pain, chronic pain, neuropathic pain, hyperalgesia, allodynia or cancer pain, including diabetic neuropathy or diabetic peripheral neuropathy and osteoarthritis or fibromyalgia.

Fig. 1)

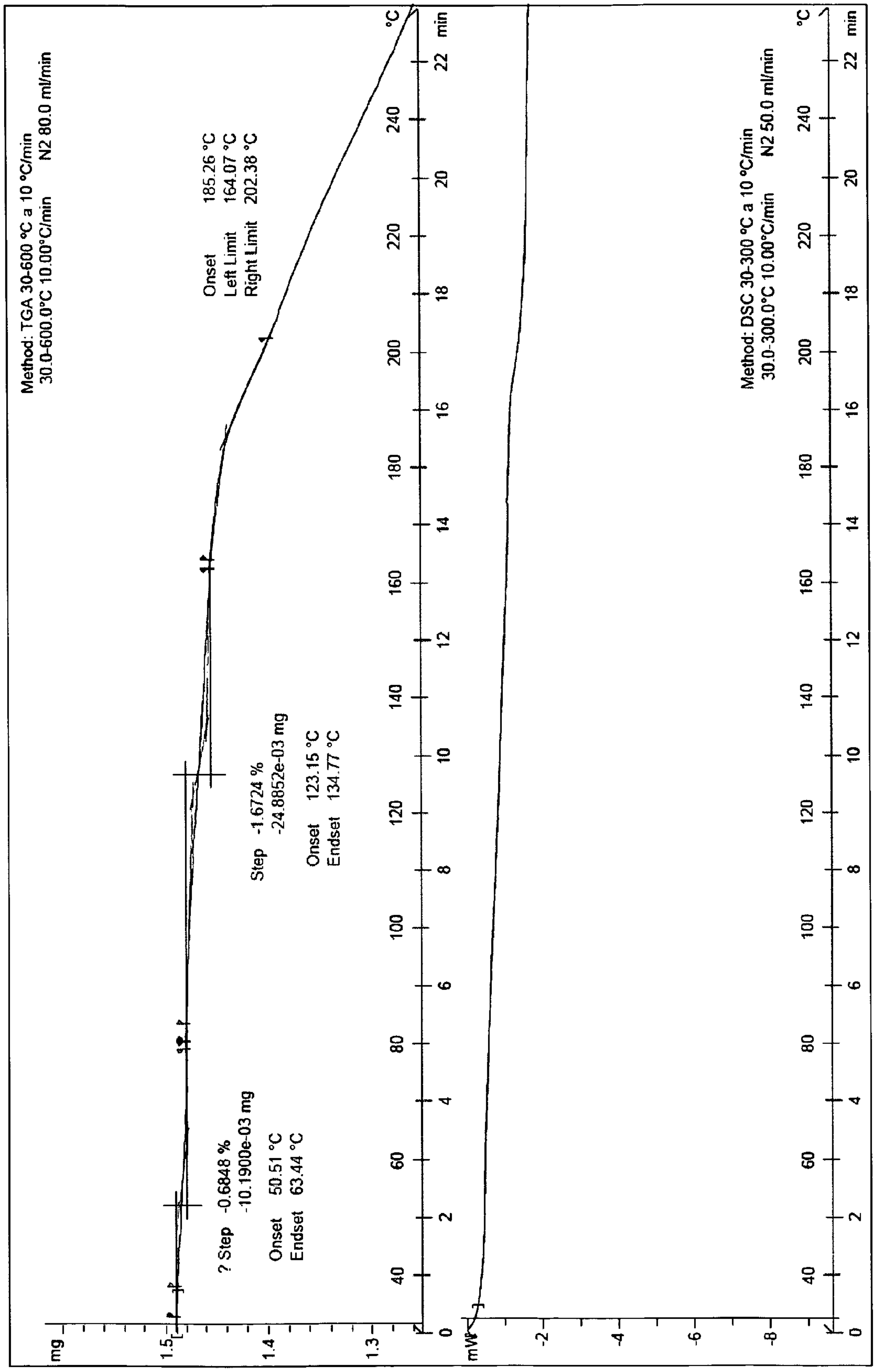
Method: TGA 30-600 °C a 10 °C/min
30.0-600.0°C 10.00°C/min N2 80.0 ml/min
Onset 185.26 °C
Left Limit 164.07 °C
Right Limit 202.38 °C
Step -1.6724 %
-24.8852e-03 mg
Onset 123.15 °C
Endset 134.77 °C
? Step -0.6848 %
-10.1900e-03 mg
Onset 50.51 °C
Endset 63.44 °C
mg
1.5
1.4
1.3
0
40
60
80
100
120
140
160
180
200
220
240
°C
2
4
6
8
10
12
14
16
18
20
22
min
Method: DSC 30-300 °C a 10 °C/min
30.0-300.0°C 10.00°C/min N2 50.0 ml/min
mW
0
-2
-4
-6
-8

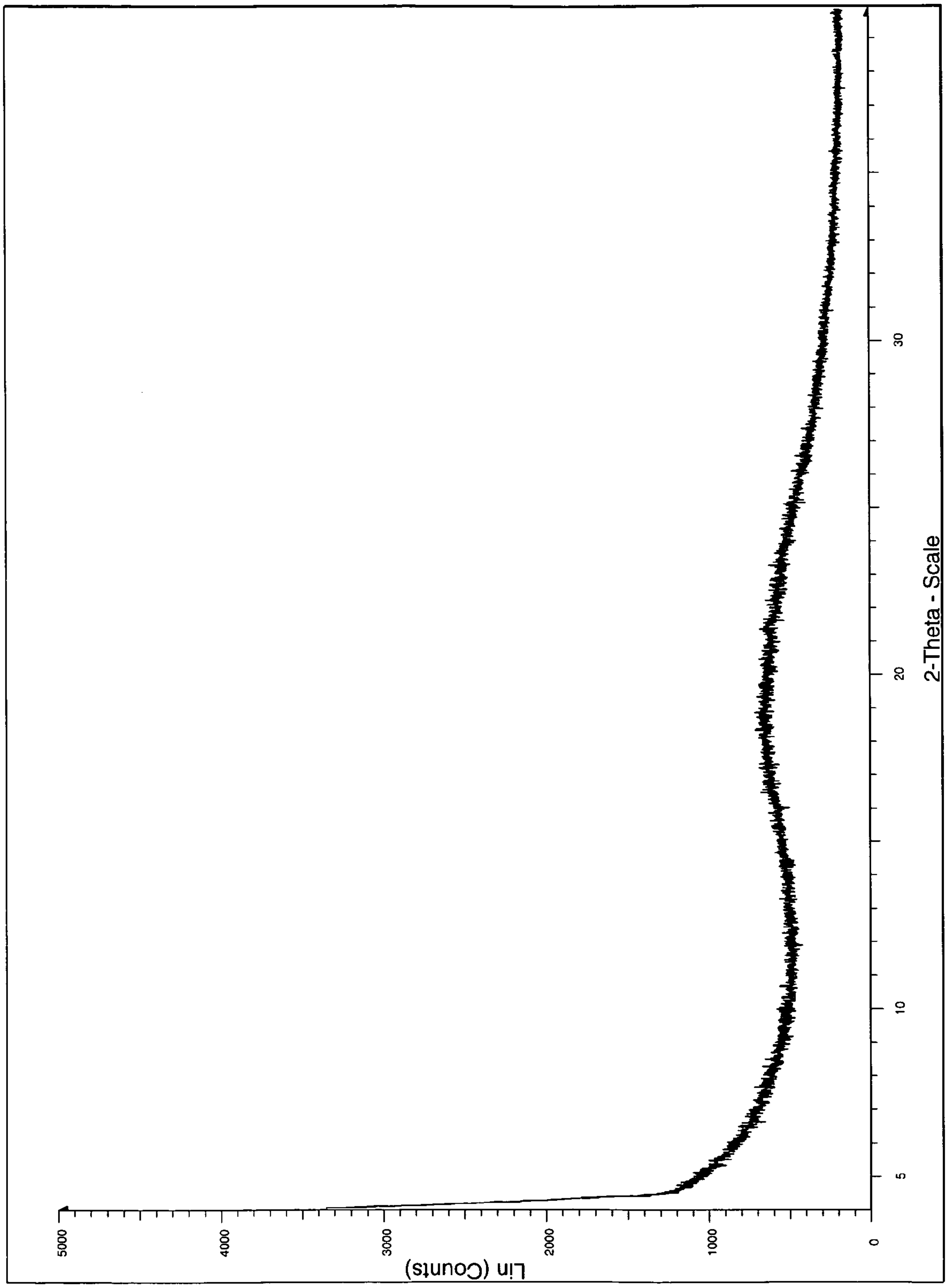


Fig. 2)

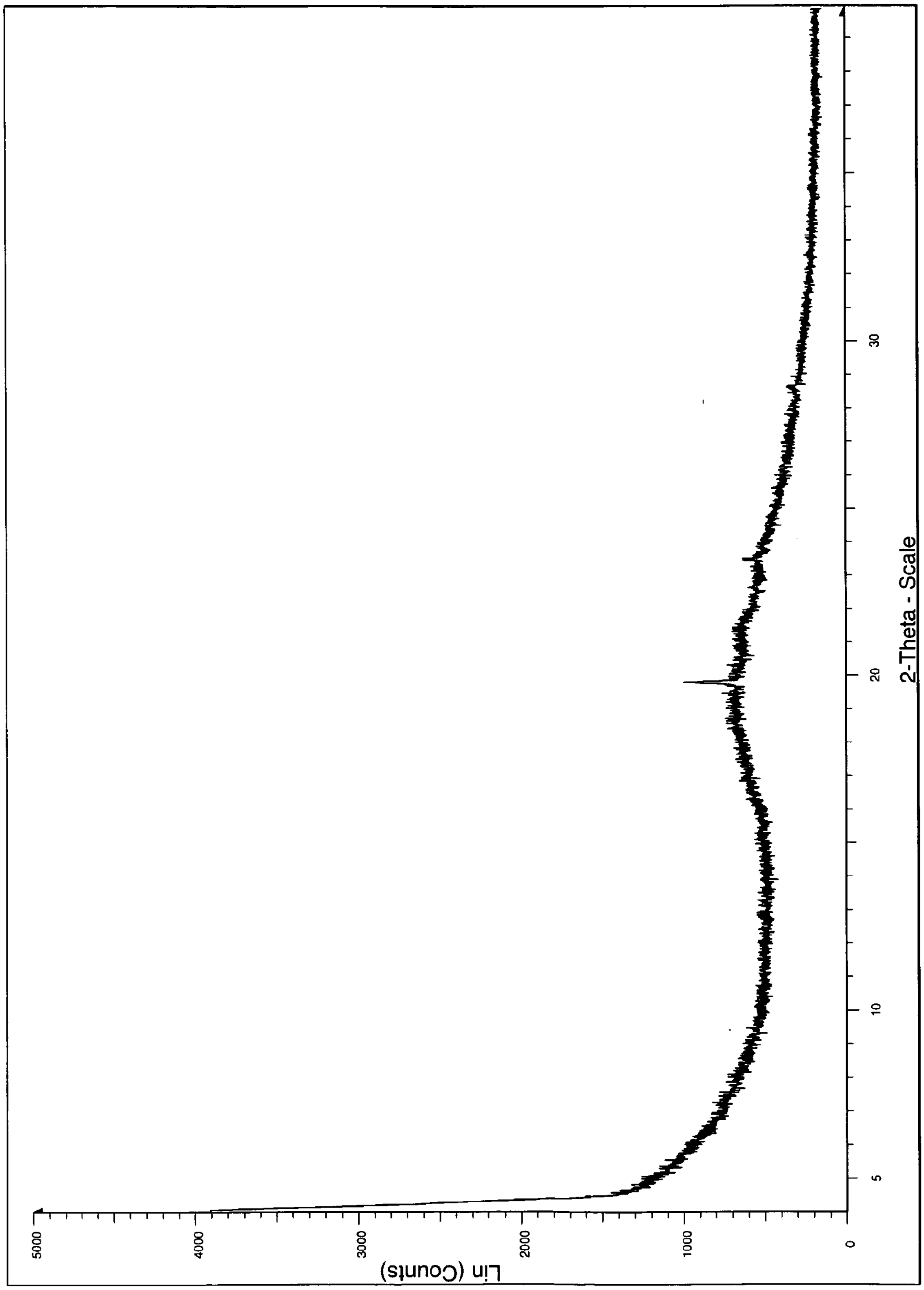


Fig. 3)

Fig. 4)

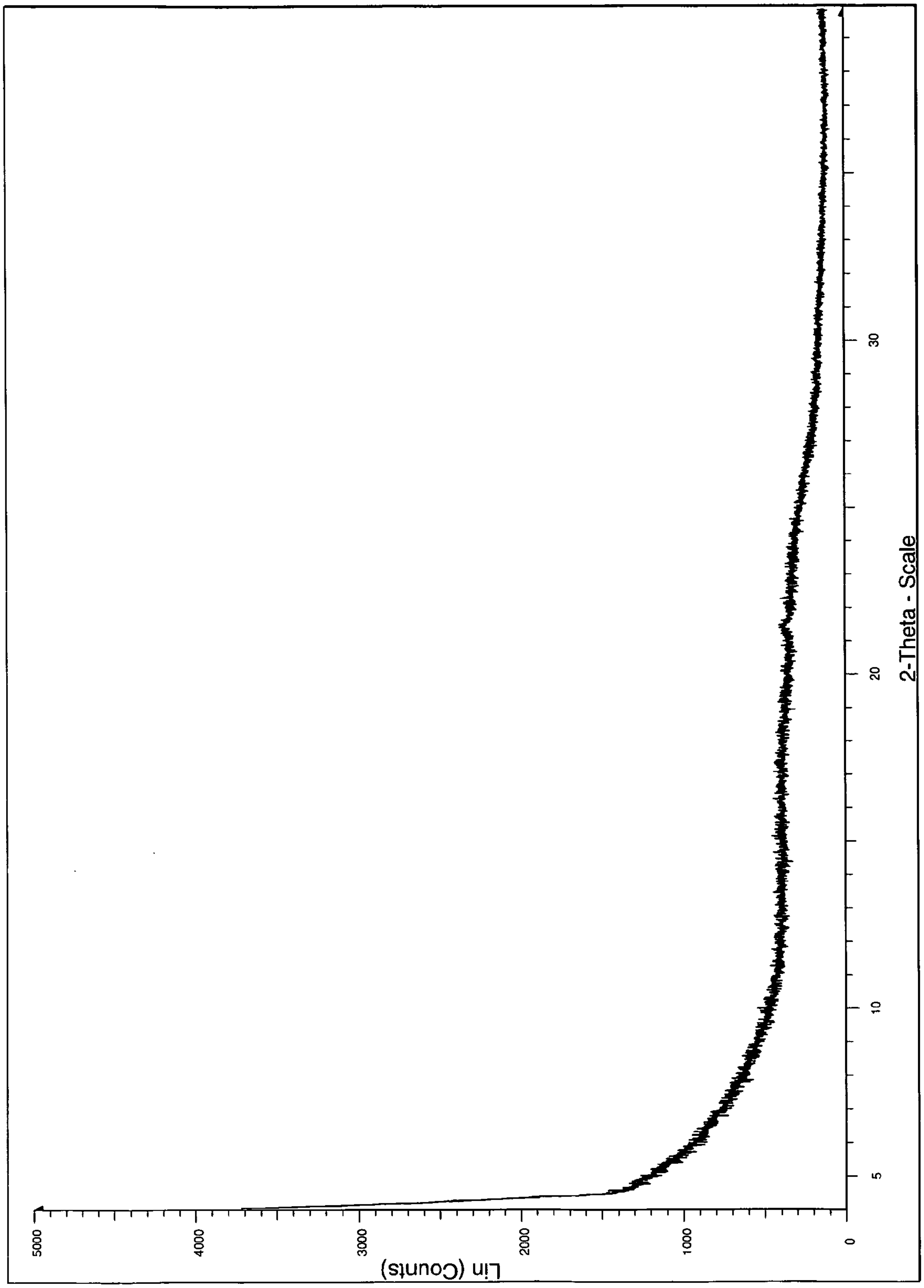

Fig. 5)

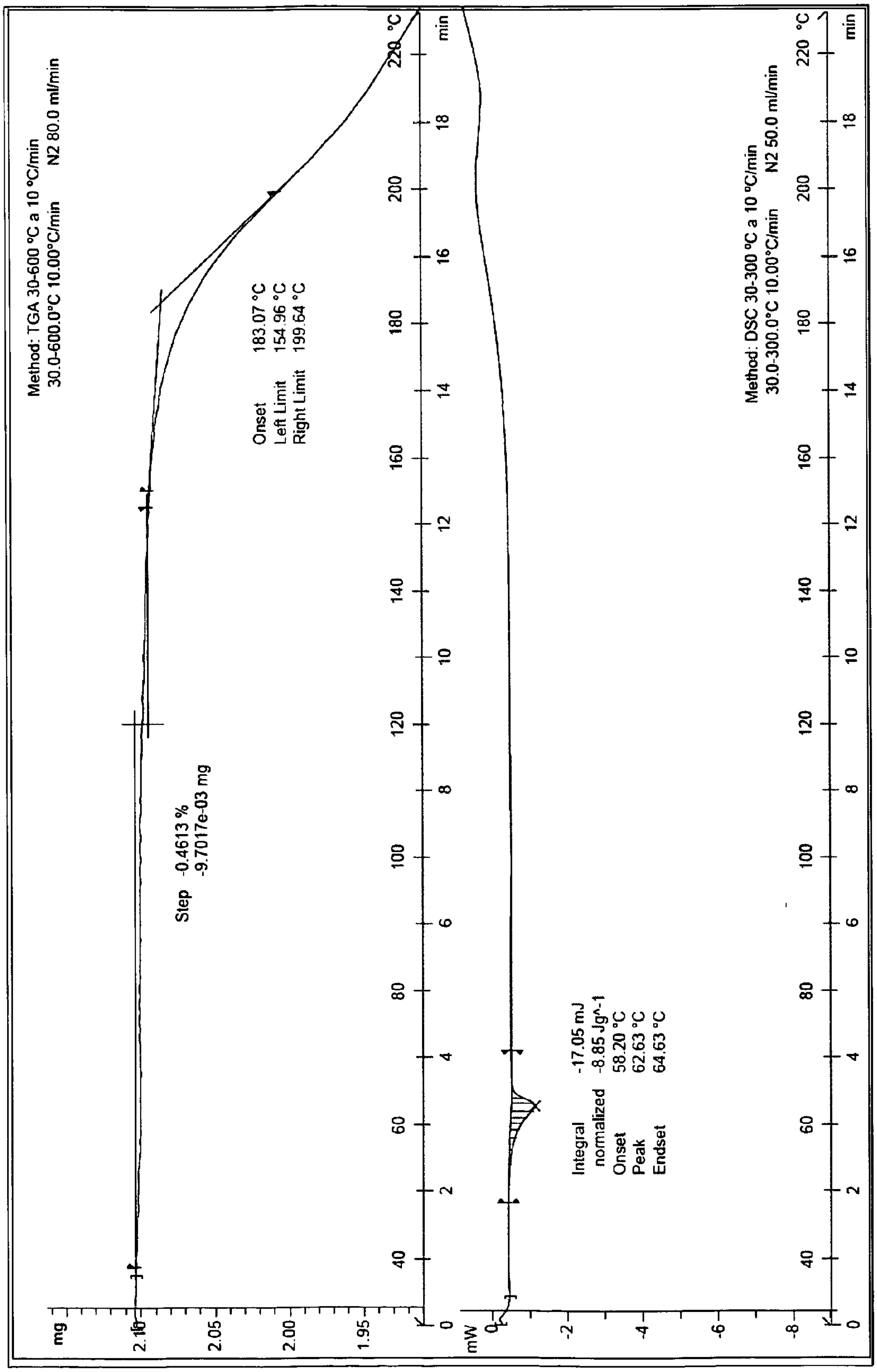
Method: TGA 30-600 °C a 10 °C/min
30.0-600.0°C 10.00°C/min N2 80.0 ml/min
Onset 183.07 °C
Left Limit 154.96 °C
Right Limit 199.64 °C
Step -0.4613 %
-9.7017e-03 mg
mg
2.10
2.05
2.00
1.95
40
60
80
100
120
140
160
180
200
220 °C
0
2
4
6
8
10
12
14
16
18
min
Method: DSC 30-300 °C a 10 °C/min
30.0-300.0°C 10.00°C/min N2 50.0 ml/min
Integral -17.05 mJ
normalized -8.85 Jg^-1
Onset 58.20 °C
Peak 62.63 °C
Endset 64.63 °C
mW
0
-2
-4
-6
-8

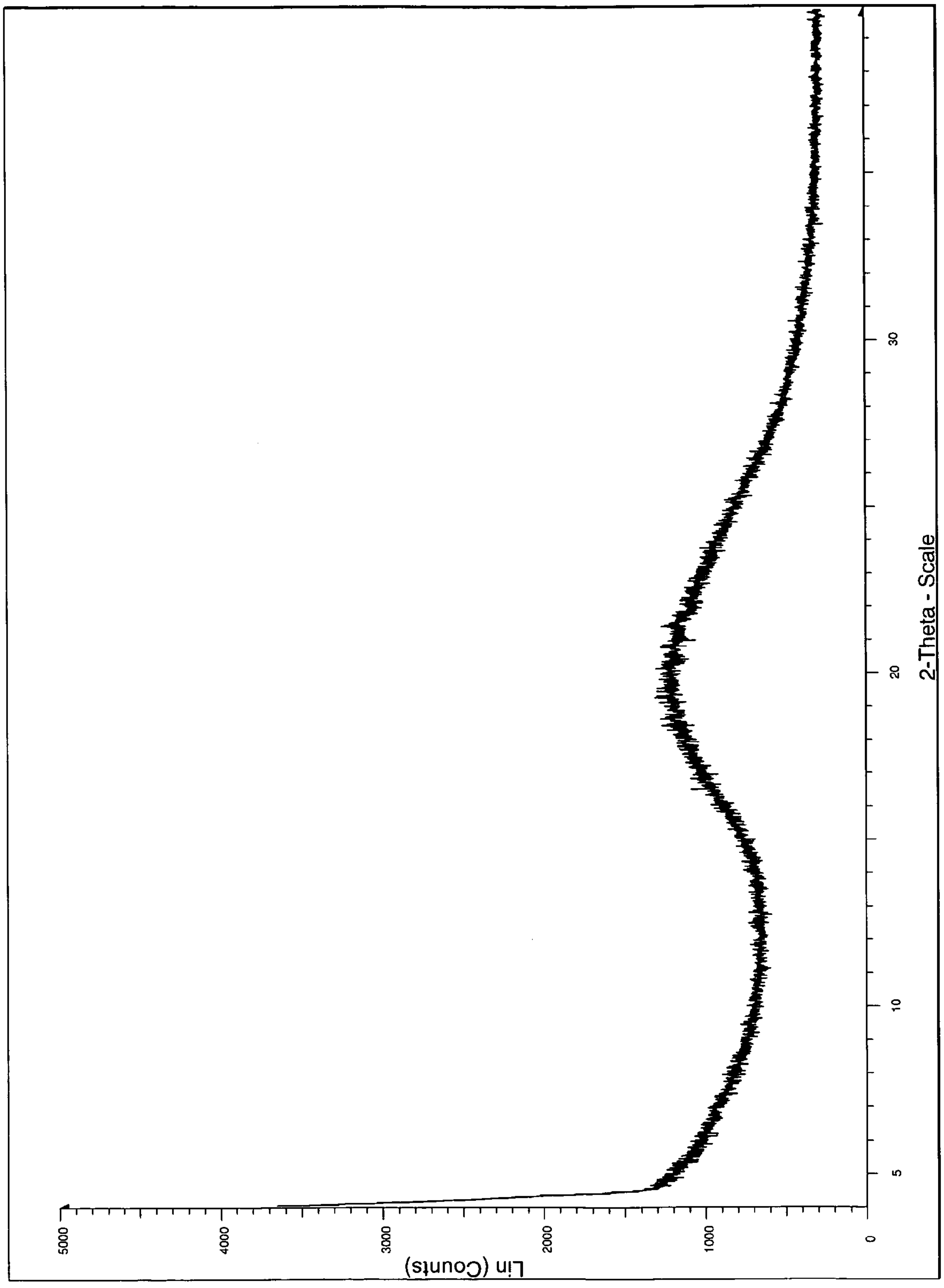


Fig. 6)

Fig. 7)

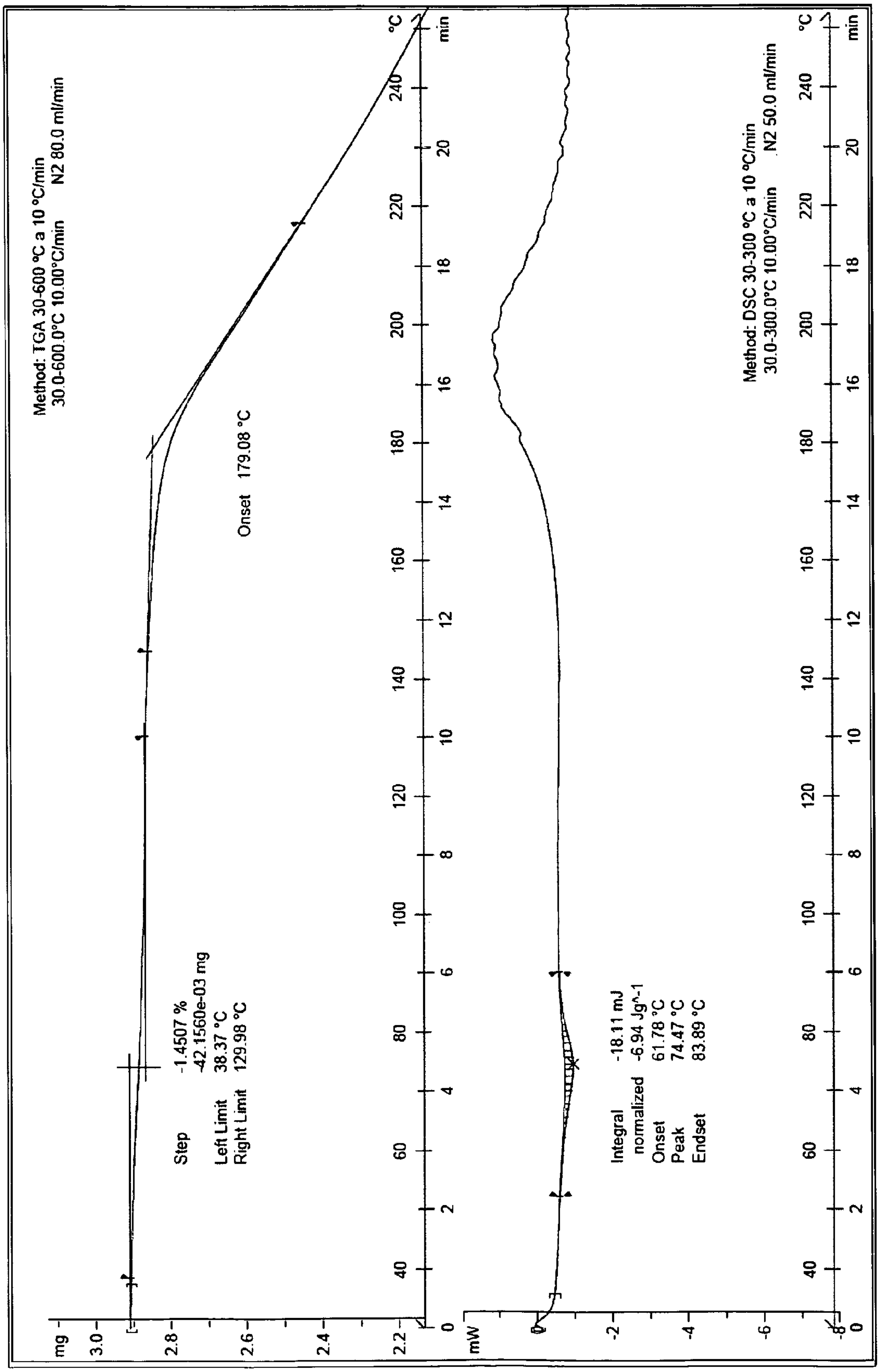
Method: TGA 30-600 ºC a 10 ºC/min
30.0-600.0°C 10.00°C/min N2 80.0 ml/min
Onset 179.08 °C
Step -1.4507 %
-42.1560e-03 mg
Left Limit 38.37 °C
Right Limit 129.98 °C
mg
°C
min
Method: DSC 30-300 ºC a 10 ºC/min
30.0-300.0°C 10.00°C/min N2 50.0 ml/min
Integral -18.11 mJ
normalized -6.94 Jg^-1
Onset 61.78 °C
Peak 74.47 °C
Endset 83.89 °C
mW

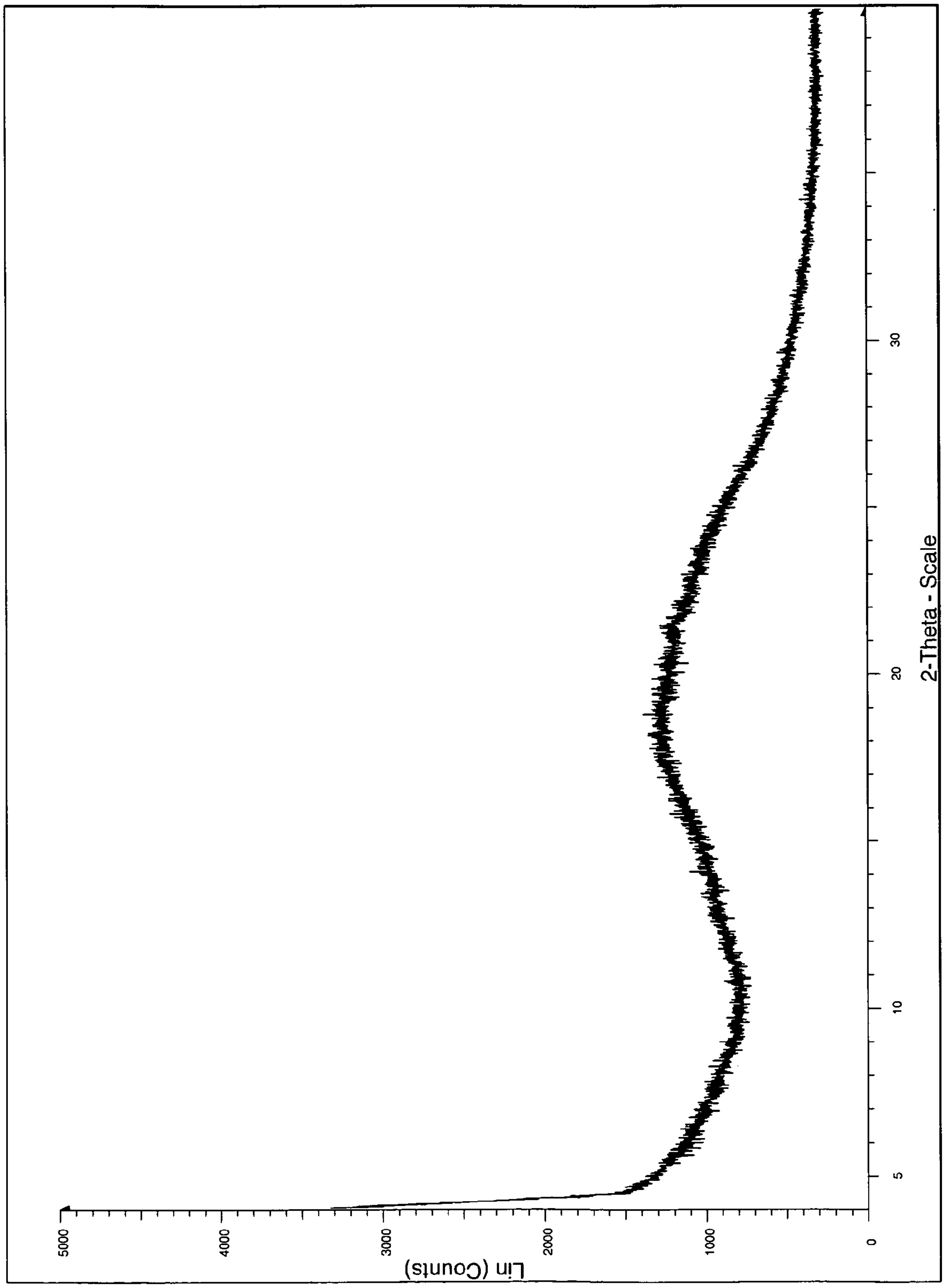


Fig. 8)

Fig. 9)

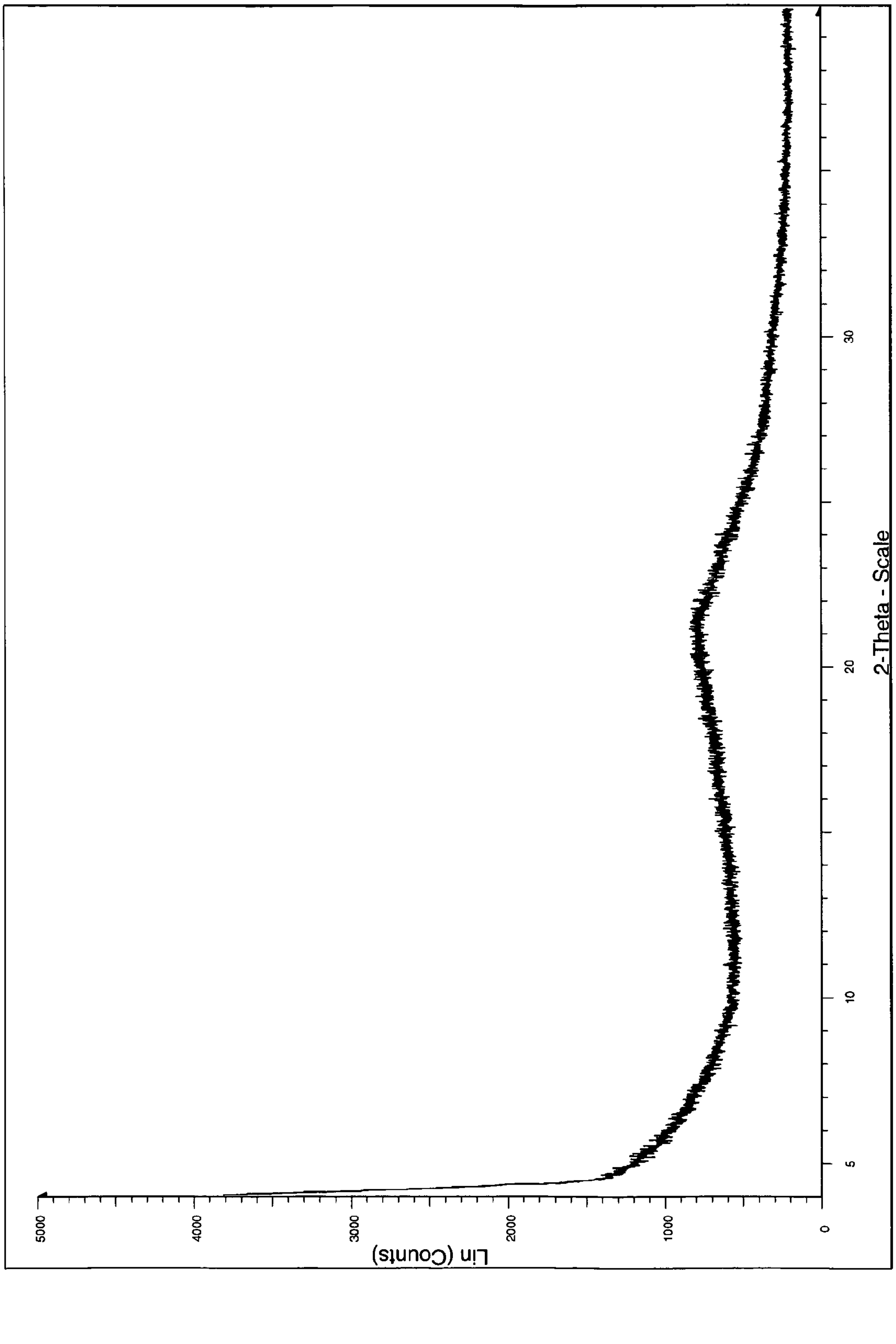

Fig. 10)

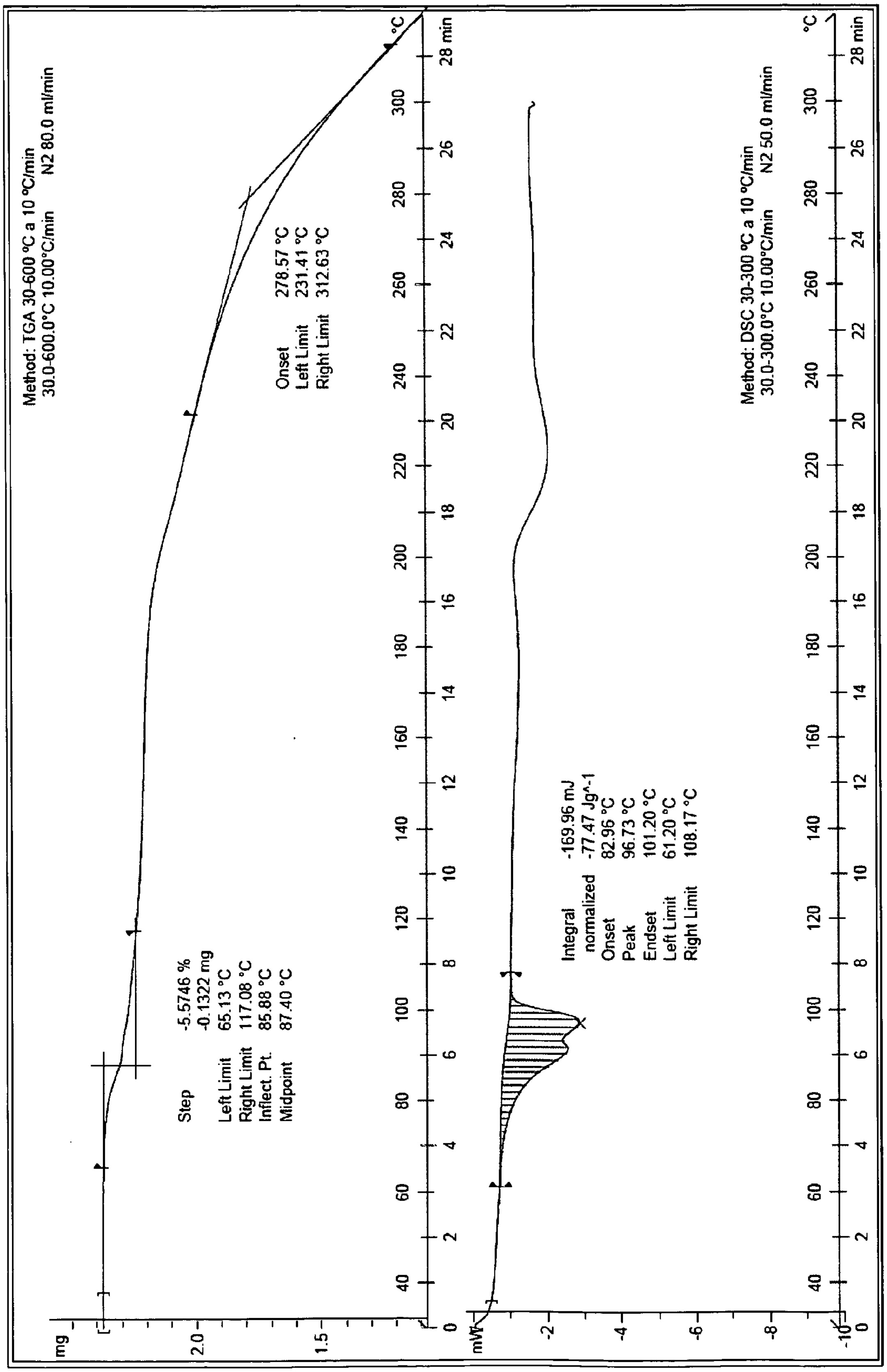
Method: TGA 30-600 ºC a 10 ºC/min
30.0-600.0°C 10.00°C/min N2 80.0 ml/min
Onset 278.57 °C
Left Limit 231.41 °C
Right Limit 312.63 °C
Step -5.5746 %
-0.1322 mg
Left Limit 65.13 °C
Right Limit 117.08 °C
Inflect. Pt. 85.88 °C
Midpoint 87.40 °C
mg
2.0
1.5
Method: DSC 30-300 ºC a 10 ºC/min
30.0-300.0°C 10.00°C/min N2 50.0 ml/min
Integral -169.96 mJ
normalized -77.47 Jg^-1
Onset 82.96 °C
Peak 96.73 °C
Endset 101.20 °C
Left Limit 61.20 °C
Right Limit 108.17 °C
mW
-2
-4
-6
-8
-10
0 40 60 80 100 120 140 160 180 200 220 240 260 280 300 °C
0 2 4 6 8 10 12 14 16 18 20 22 24 26 28 min

Fig. 11)

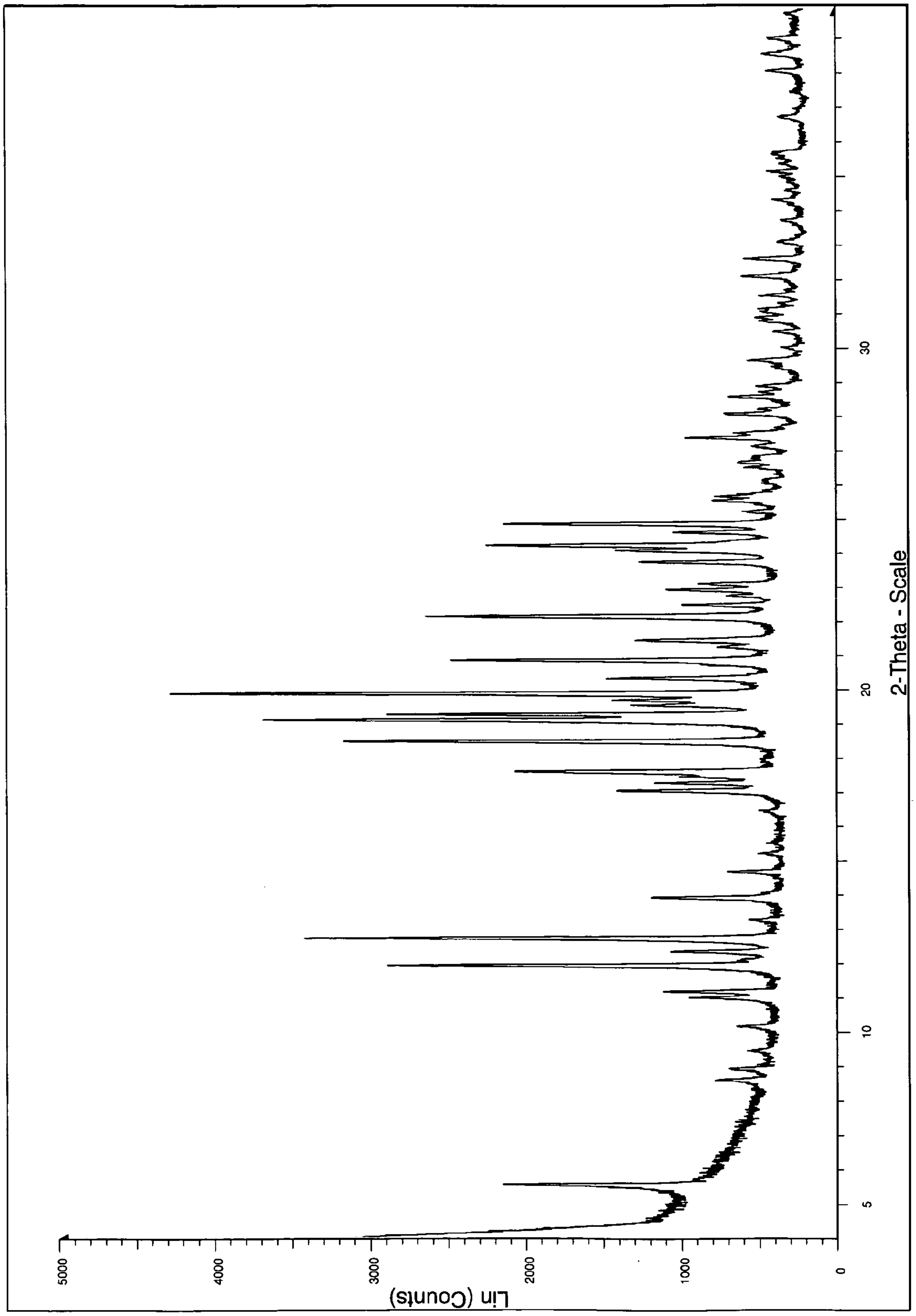

Fig. 12)

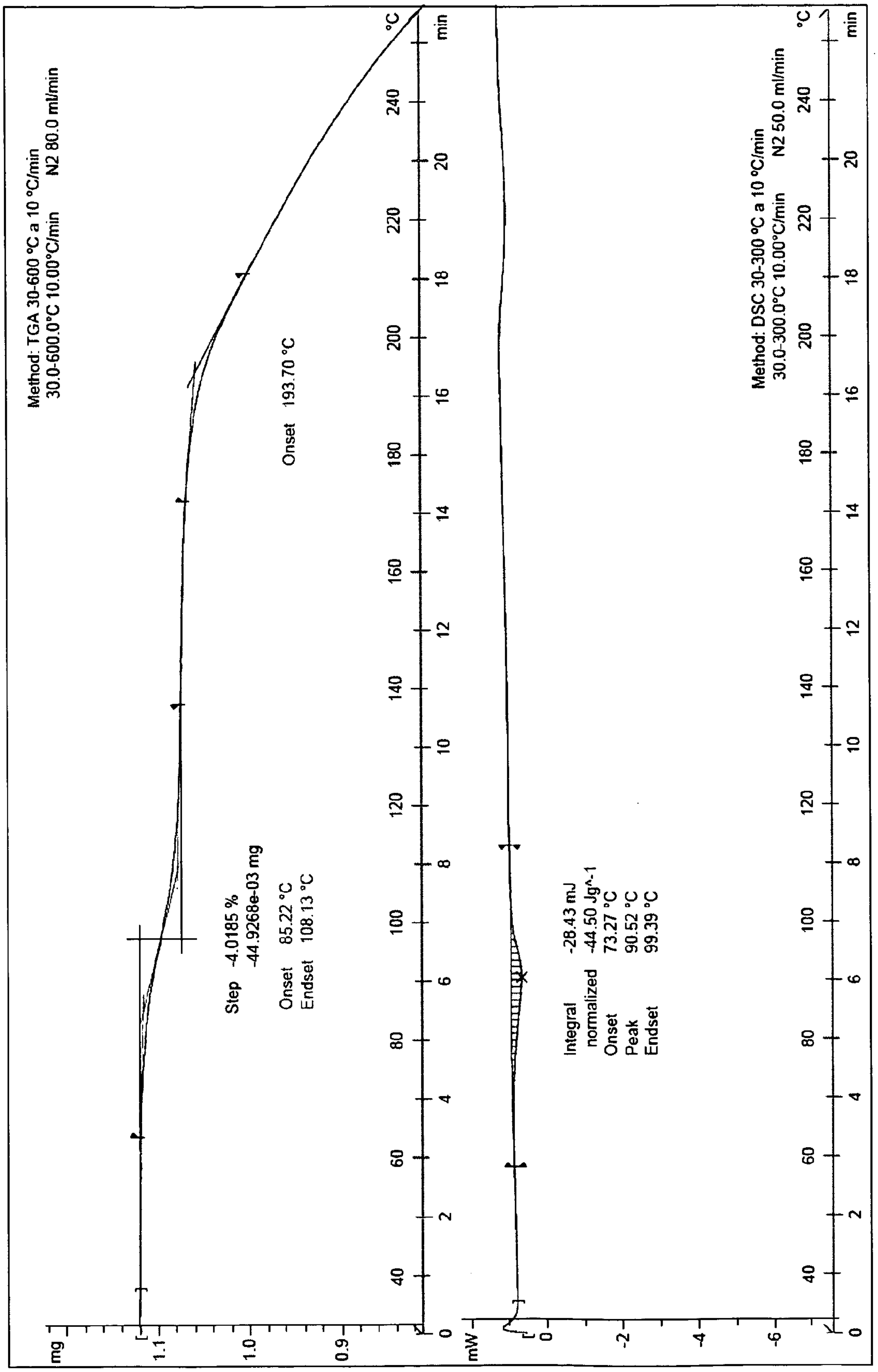
Method: TGA 30-600 °C a 10 °C/min
30.0-600.0°C 10.00°C/min N2 80.0 ml/min
Onset 193.70 °C
Step -4.0185 %
-44.9268e-03 mg
Onset 85.22 °C
Endset 108.13 °C
mg
°C
min
Method: DSC 30-300 °C a 10 °C/min
30.0-300.0°C 10.00°C/min N2 50.0 ml/min
Integral -28.43 mJ
normalized -44.50 Jg^-1
Onset 73.27 °C
Peak 90.52 °C
Endset 99.39 °C
mW
°C
min

Fig. 13)

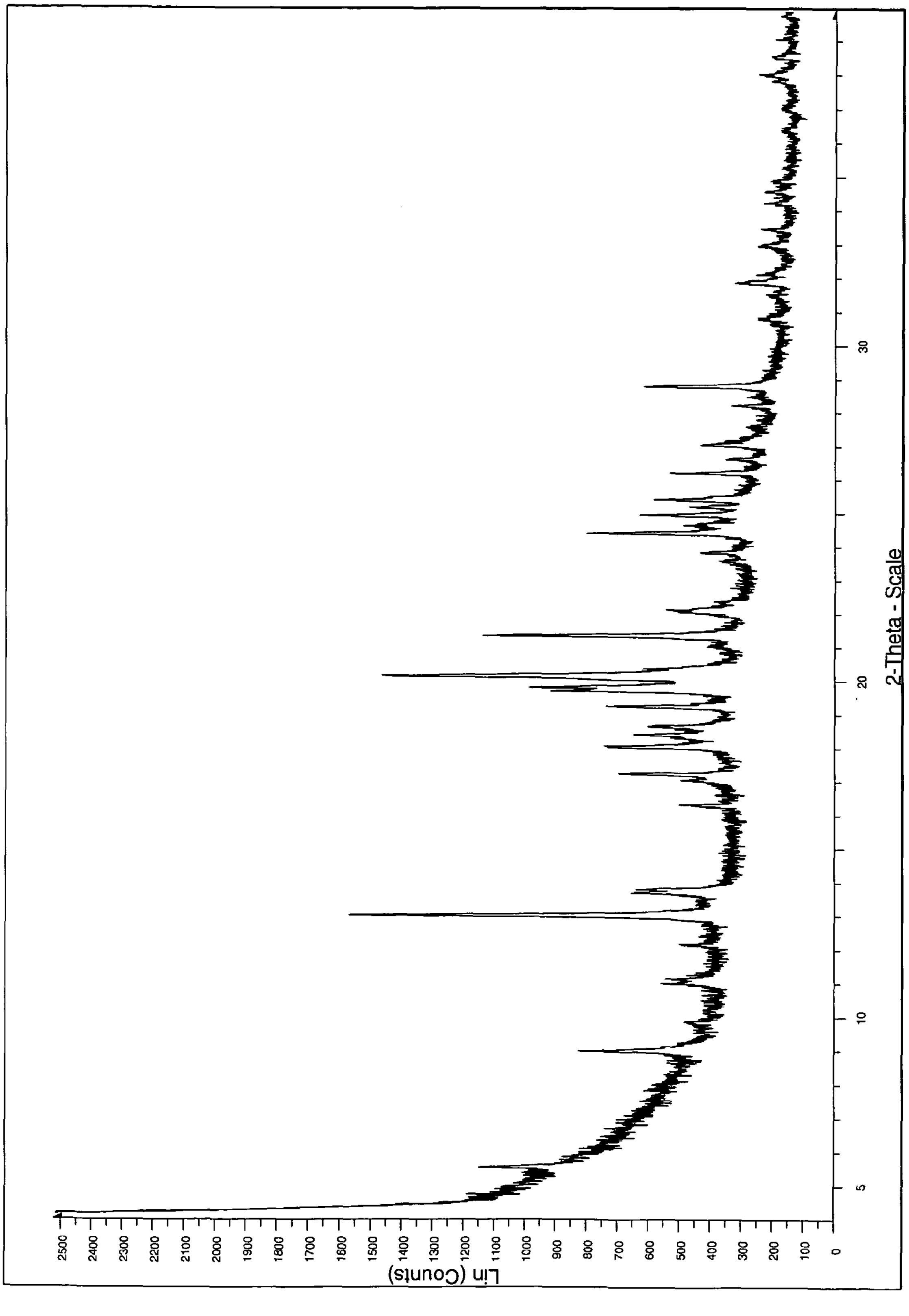
Lin (Counts)
0
100
200
300
400
500
600
700
800
900
1000
1100
1200
1300
1400
1500
1600
1700
1800
1900
2000
2100
2200
2300
2400
2500
5
10
20
30
2-Theta - Scale

Fig. 14)

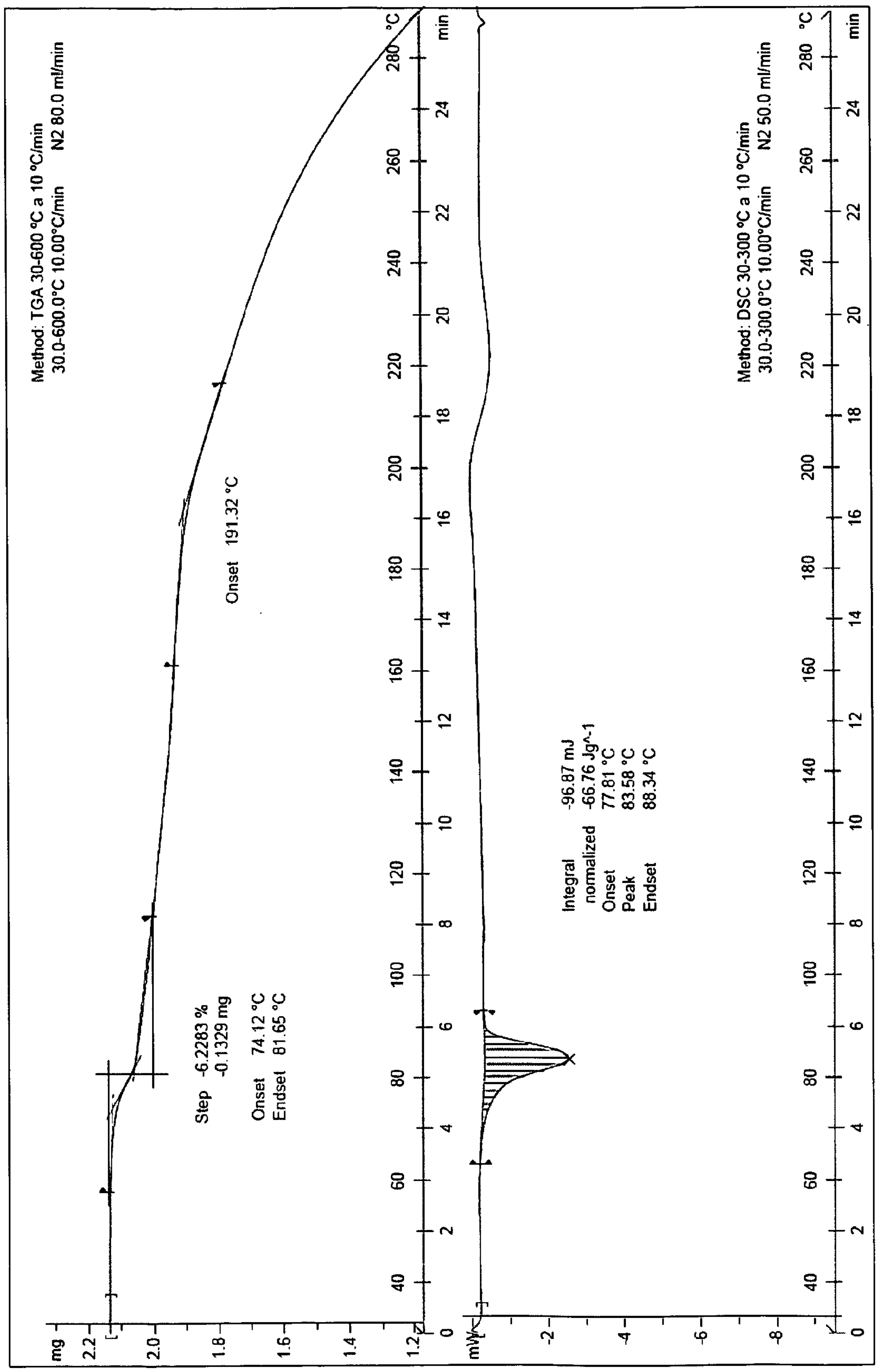
Method: TGA 30-600 °C a 10 °C/min
30.0-600.0°C 10.00°C/min N2 80.0 ml/min
Step -6.2283 %
-0.1329 mg
Onset 74.12 °C
Endset 81.65 °C
Onset 191.32 °C
Method: DSC 30-300 °C a 10 °C/min
30.0-300.0°C 10.00°C/min N2 50.0 ml/min
Integral -96.87 mJ
normalized -66.76 Jg^-1
Onset 77.81 °C
Peak 83.58 °C
Endset 88.34 °C
mg
mW
°C
min

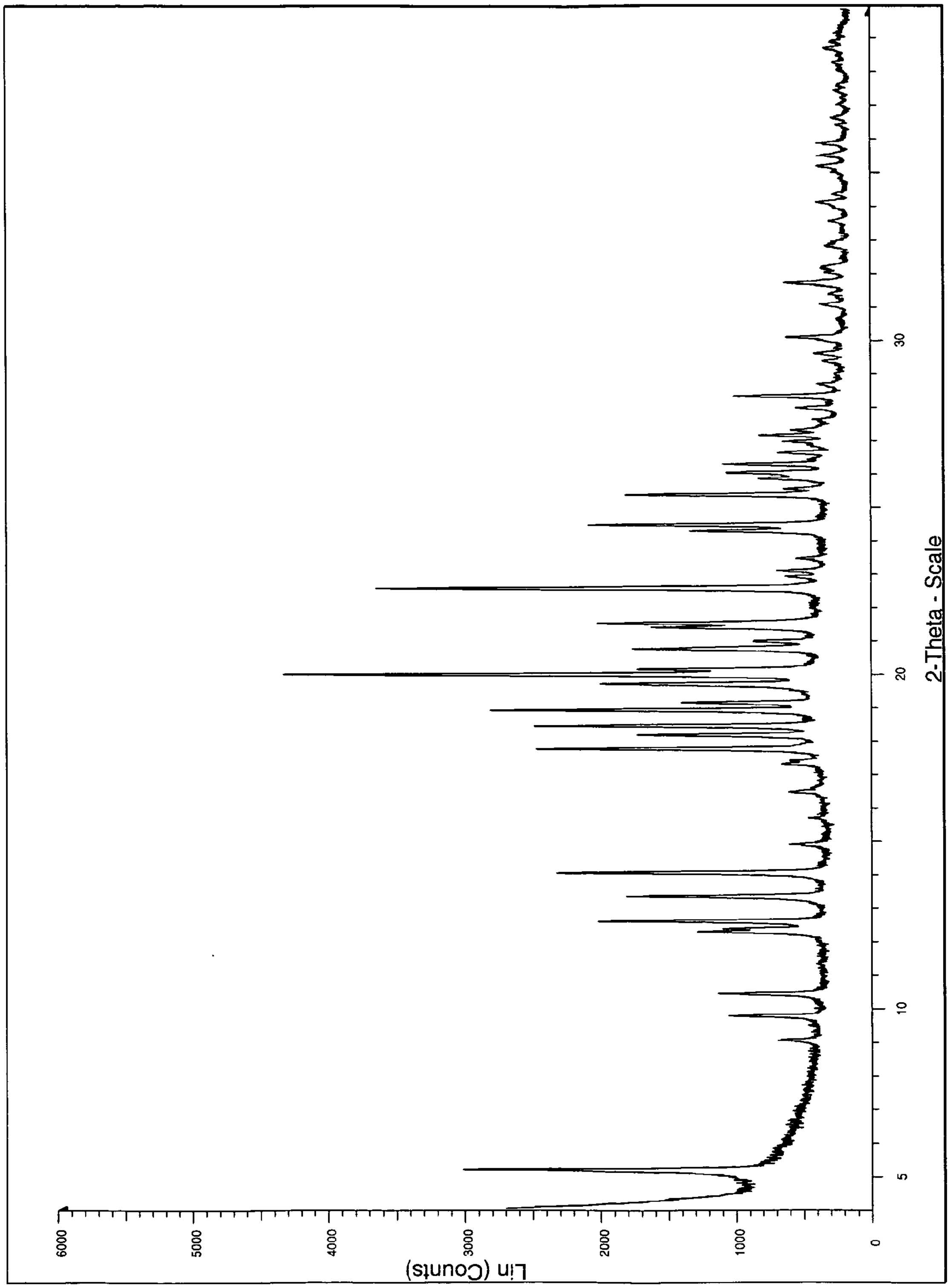
Lin (Counts)
0
1000
2000
3000
4000
5000
6000
5
10
20
30
2-Theta - Scale

Fig. 15)

Fig. 16)

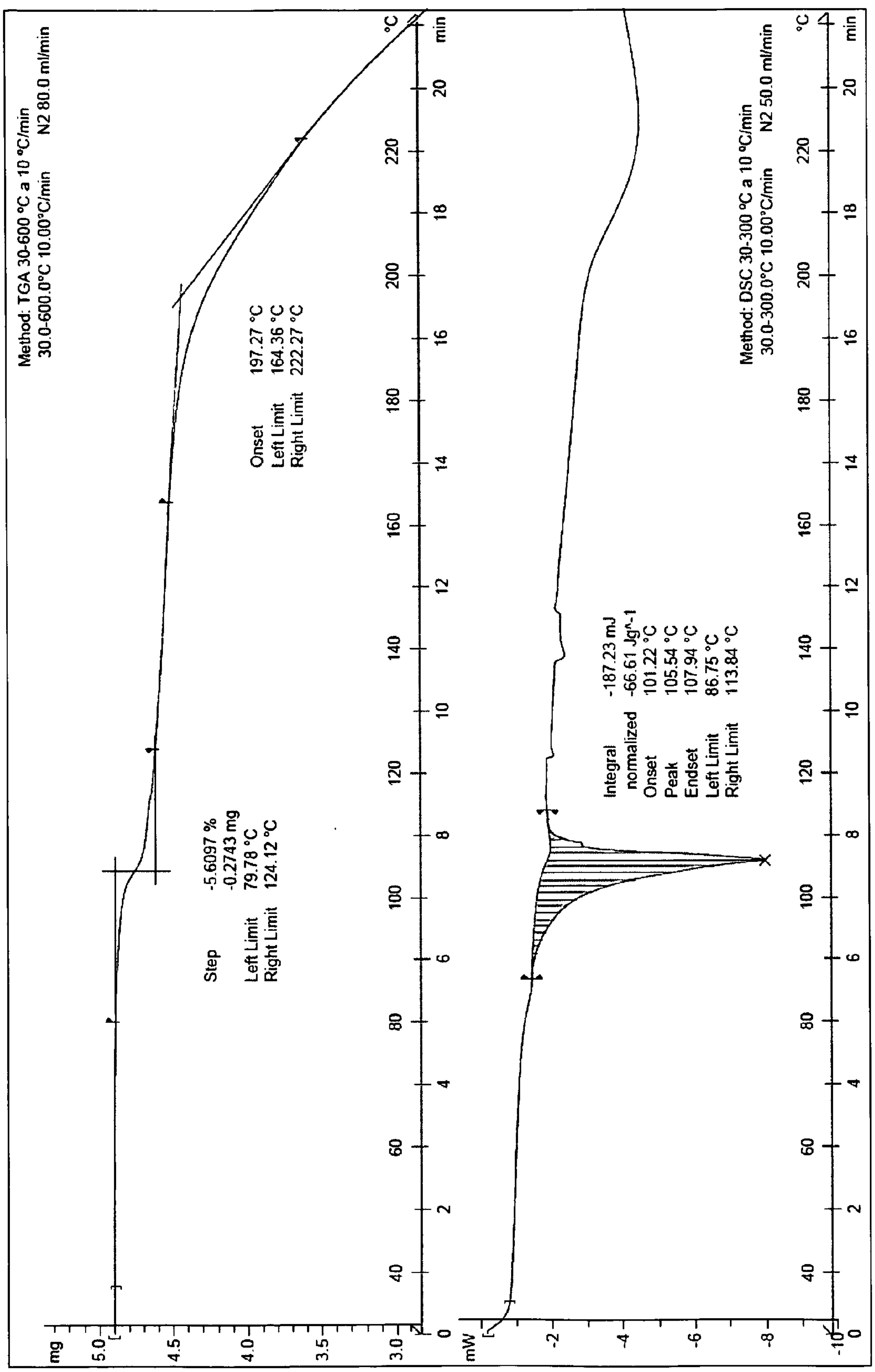
Method: TGA 30-600 °C a 10 °C/min
30.0-600.0°C 10.00°C/min N2 80.0 ml/min
Onset 197.27 °C
Left Limit 164.36 °C
Right Limit 222.27 °C
Step -5.6097 %
-0.2743 mg
Left Limit 79.78 °C
Right Limit 124.12 °C
mg
°C
min
Method: DSC 30-300 °C a 10 °C/min
30.0-300.0°C 10.00°C/min N2 50.0 ml/min
Integral -187.23 mJ
normalized -66.61 Jg^-1
Onset 101.22 °C
Peak 105.54 °C
Endset 107.94 °C
Left Limit 86.75 °C
Right Limit 113.84 °C
mW

Fig. 17)

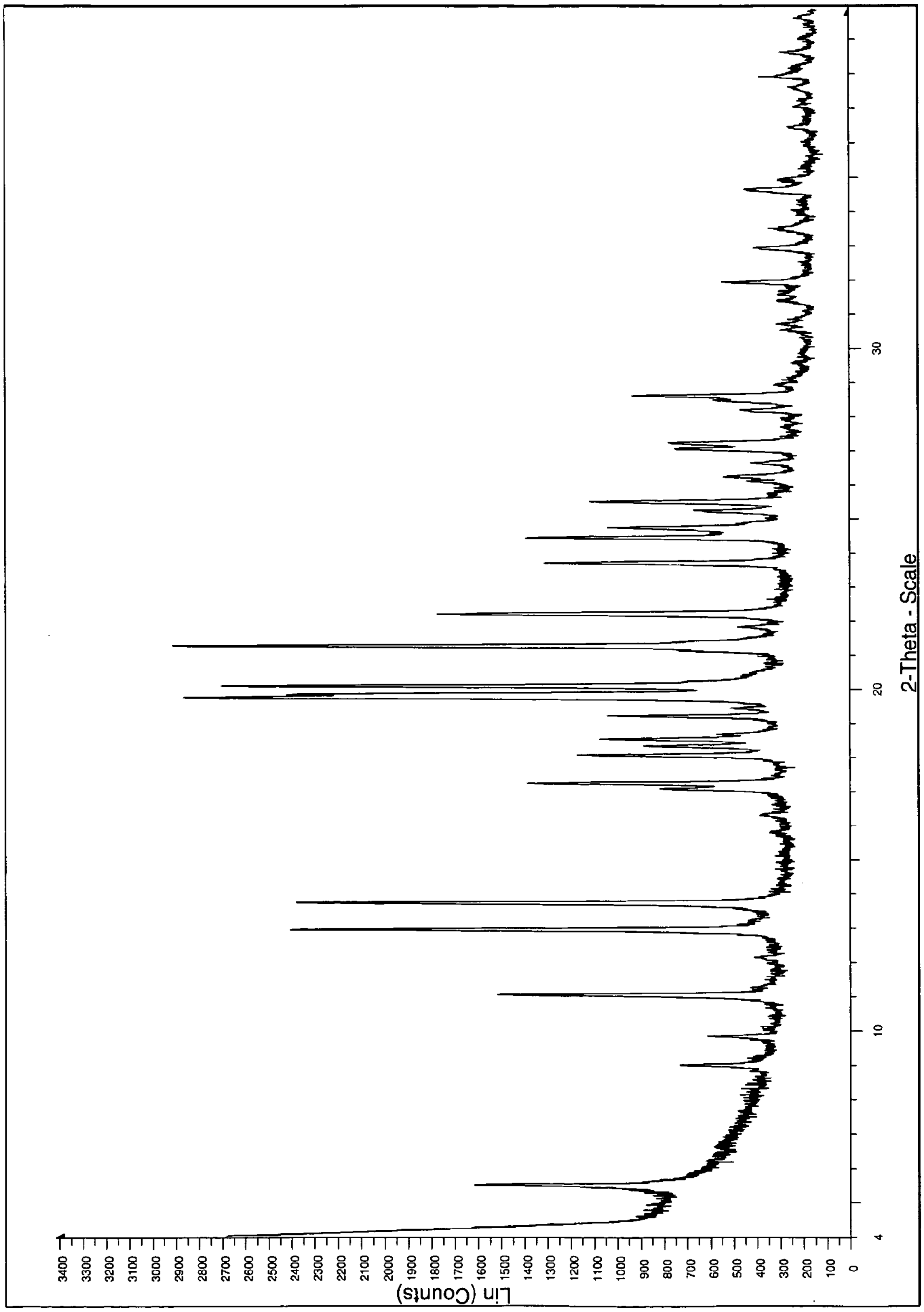
Lin (Counts)
0
100
200
300
400
500
600
700
800
900
1000
1100
1200
1300
1400
1500
1600
1700
1800
1900
2000
2100
2200
2300
2400
2500
2600
2700
2800
2900
3000
3100
3200
3300
3400
4
10
20
30
2-Theta - Scale

Fig 18)

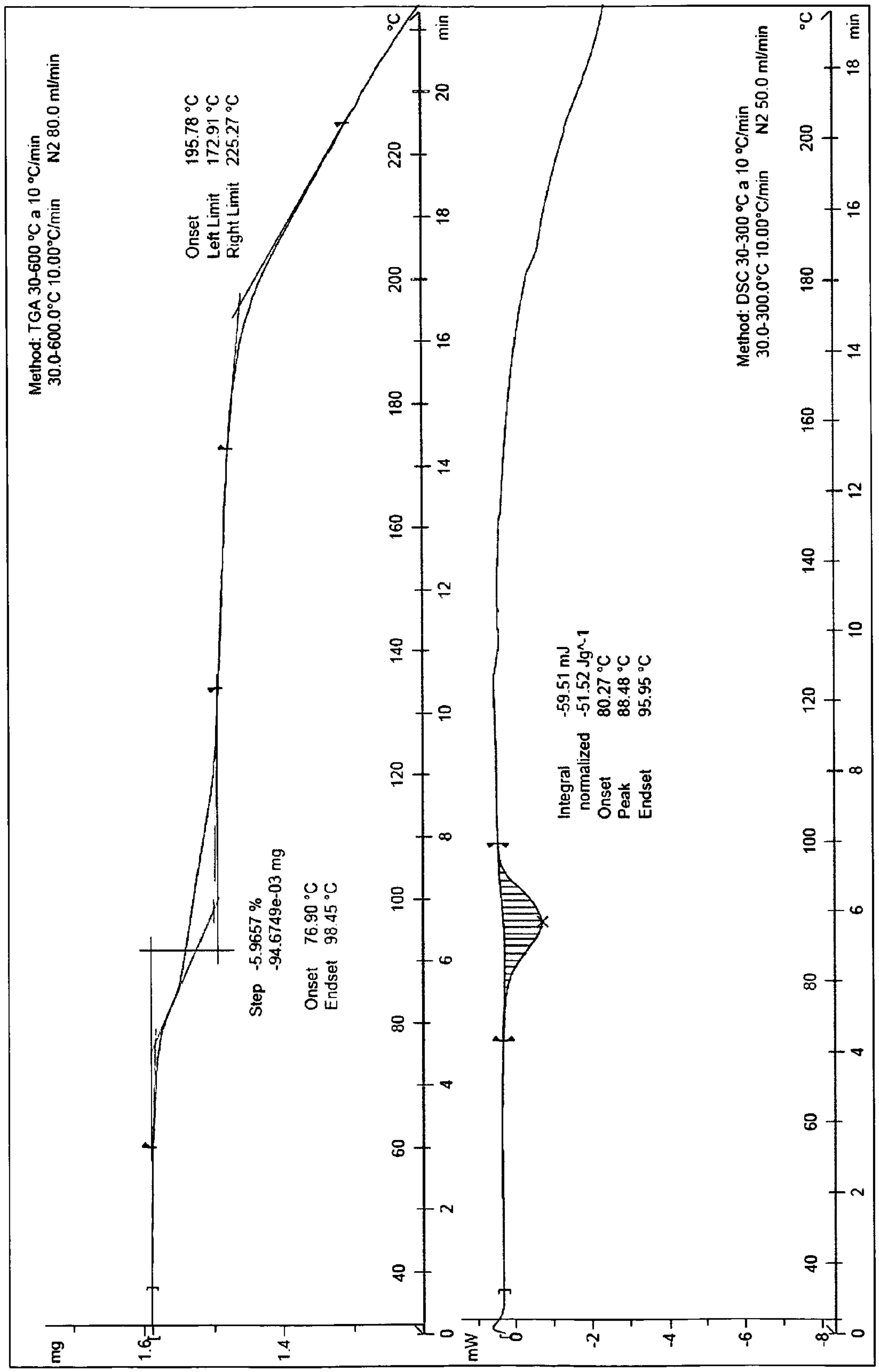
Method: TGA 30-600 °C a 10 °C/min
30.0-600.0°C 10.00°C/min N2 80.0 ml/min
Onset 195.78 °C
Left Limit 172.91 °C
Right Limit 225.27 °C
Step -5.9657 %
-94.6749e-03 mg
Onset 76.90 °C
Endset 98.45 °C
Integral -59.51 mJ
normalized -51.52 Jg^-1
Onset 80.27 °C
Peak 88.48 °C
Endset 95.95 °C
Method: DSC 30-300 °C a 10 °C/min
30.0-300.0°C 10.00°C/min N2 50.0 ml/min
mg
mW
°C
min

Fig. 19)

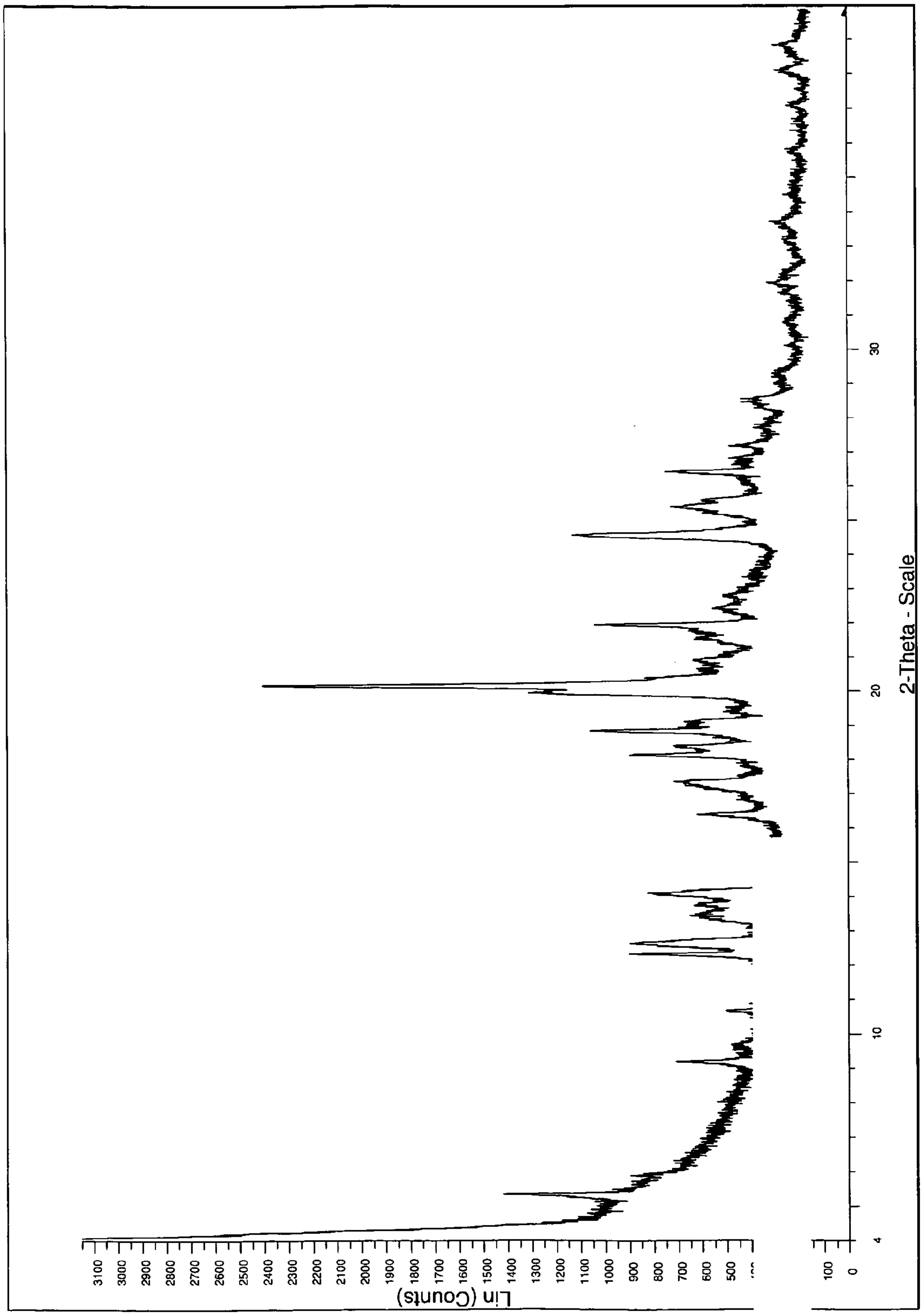
Lin (Counts)
3100
3000
2900
2800
2700
2600
2500
2400
2300
2200
2100
2000
1900
1800
1700
1600
1500
1400
1300
1200
1100
1000
900
800
700
600
500
100
0
4
10
20
30
2-Theta - Scale

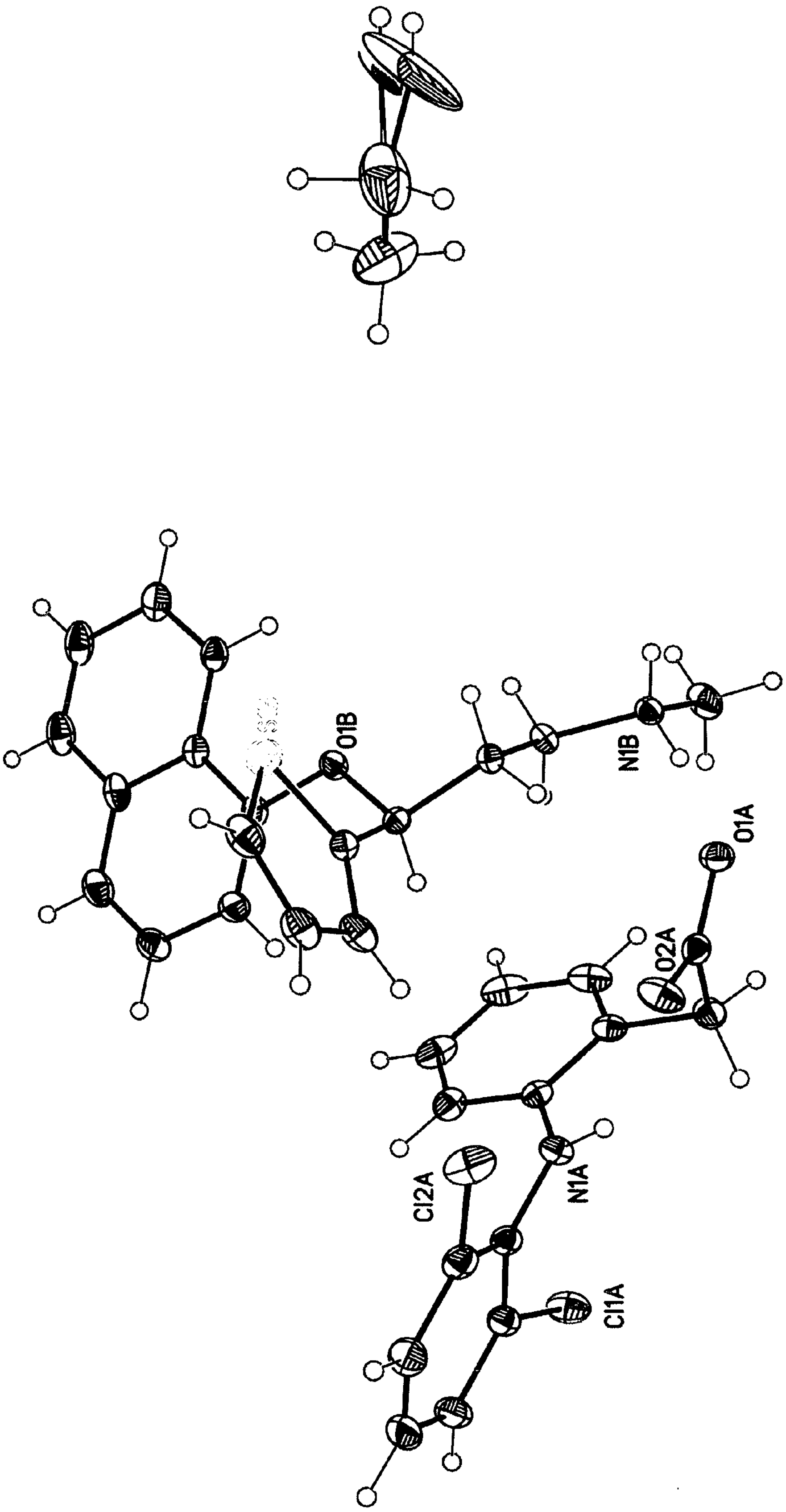

Fig. 20)

Fig. 21)

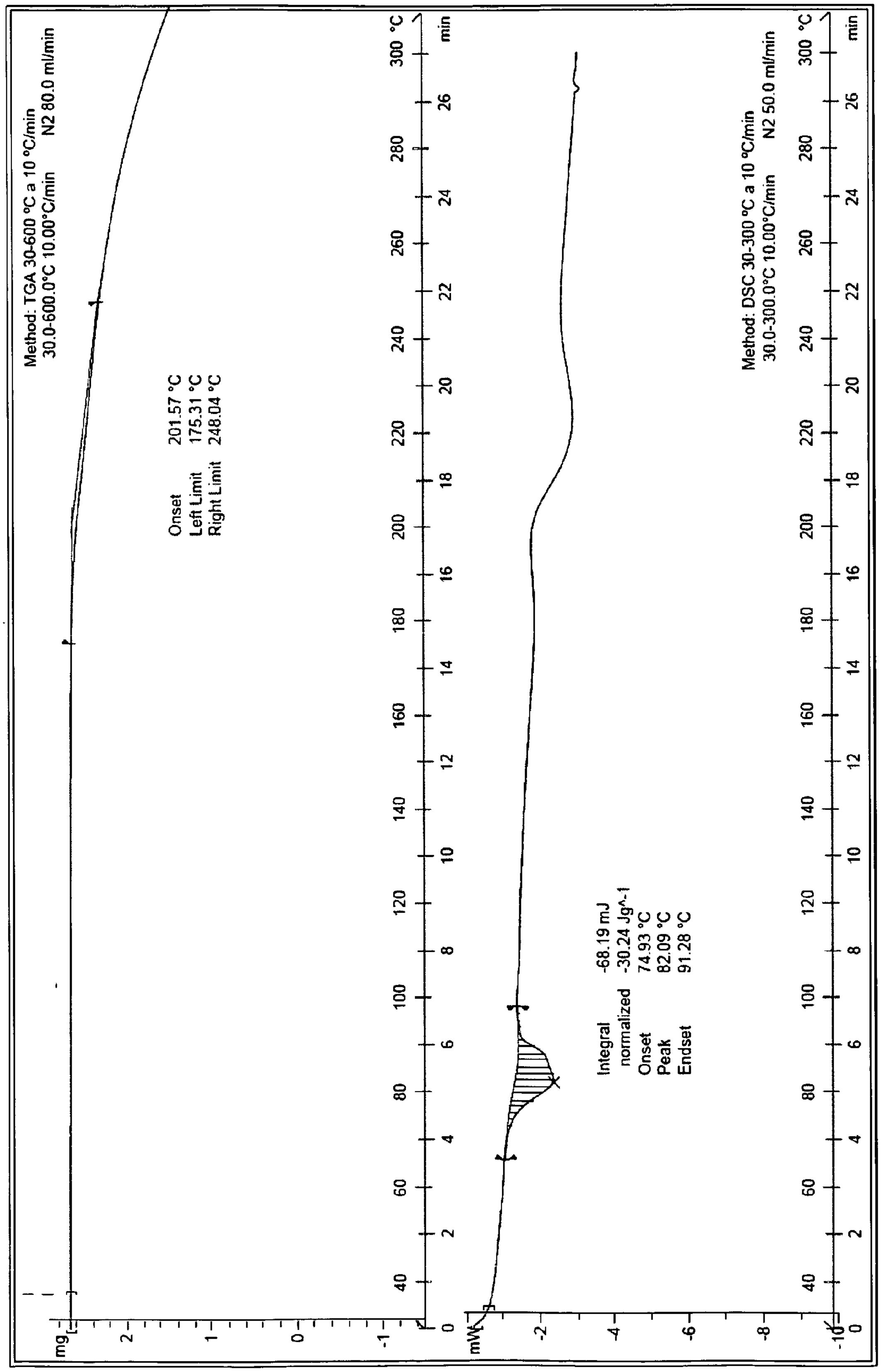
Method: TGA 30-600 °C a 10 °C/min
30.0-600.0°C 10.00°C/min N2 80.0 ml/min
Onset 201.57 °C
Left Limit 175.31 °C
Right Limit 248.04 °C
mg
Method: DSC 30-300 °C a 10 °C/min
30.0-300.0°C 10.00°C/min N2 50.0 ml/min
Integral -68.19 mJ
normalized -30.24 Jg^-1
Onset 74.93 °C
Peak 82.09 °C
Endset 91.28 °C
mW
°C
min

Fig. 22)

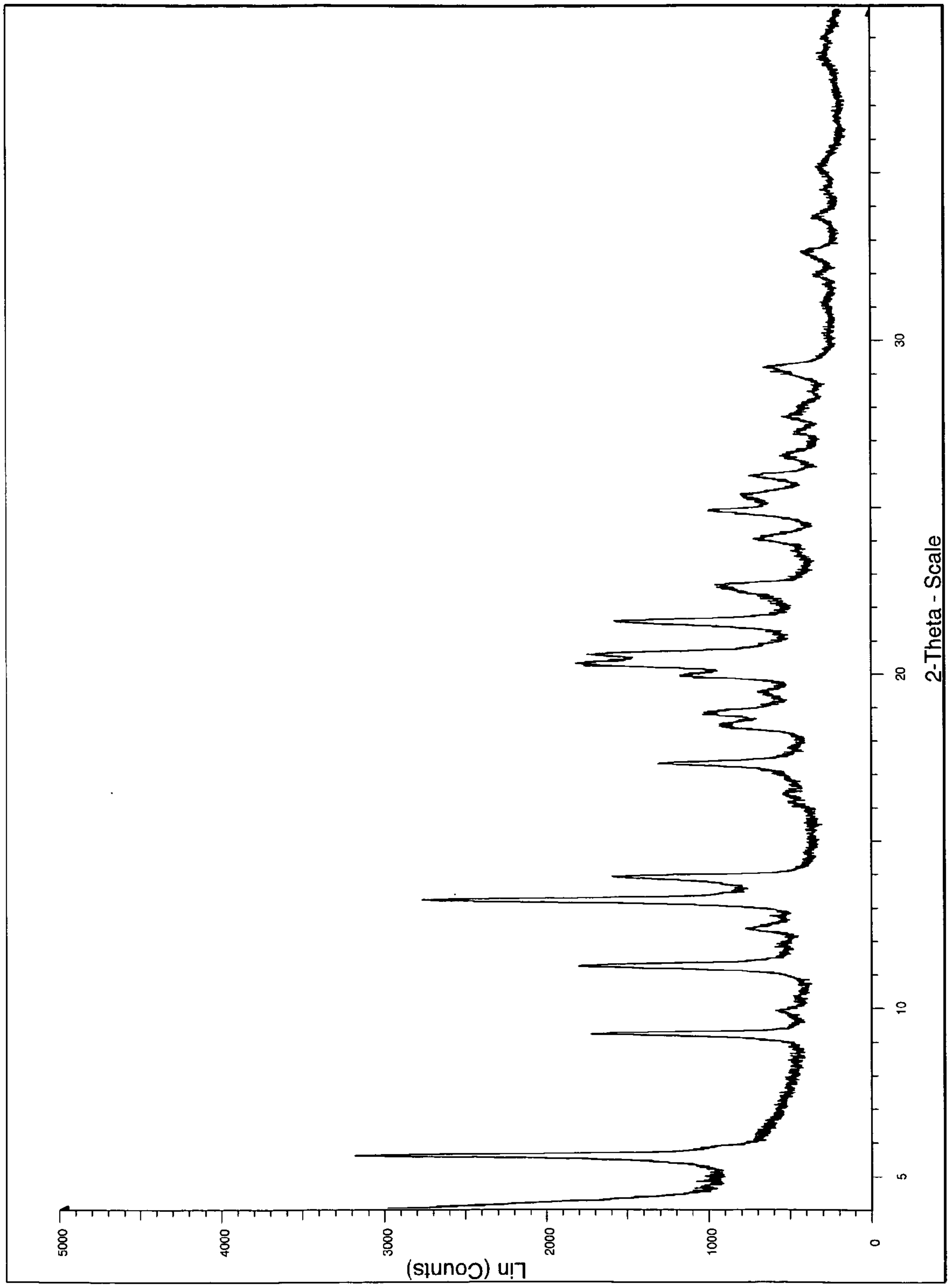

Europäisches Patentamt
European Patent Office
Office européen des brevets

# EUROPEAN SEARCH REPORT

Application Number
EP 09 38 4006

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 00/28980 A2 (LILLY CO ELI [US]; IYENGAR SMRITI [US]; JONES CARRIE KIMBERLY [US]; SH) 25 May 2000 (2000-05-25)<br>* the whole document *<br>----- | 1-15 | INV.<br>C07D333/16<br>A61K31/381<br>A61P29/00 |
| X | JONES ET AL: "Synergistic interactions between the dual serotonergic, noradrenergic reuptake inhibitor duloxetine and the non-steroidal anti-inflammatory drug ibuprofen in inflammatory pain in rodents"<br>EUROPEAN JOURNAL OF PAIN, SAUNDERS, LONDON, GB,<br>vol. 11, no. 2, 2007, pages 208-215, XP005744281<br>ISSN: 1090-3801<br>* the whole document *<br>----- | 1-15 | |
| X | WO 2007/134168 A2 (REDDYS LAB LTD DR [IN]; REDDYS LAB INC DR [US]; GUDIPATI SRINIVASALU [)<br>22 November 2007 (2007-11-22)<br>* see formula X *<br>----- | 1-2,12 | TECHNICAL FIELDS SEARCHED (IPC)<br>C07D<br>A61K<br>A61P |
| A | WO 2008/133884 A2 (COMBINATORX INC [US]; CHDI INC [US]; JIN XIAOWEI [US]; STAUNTON JANE [) 6 November 2008 (2008-11-06)<br>* page 62; claims 6,12; table 3b *<br>----- | 1-15 | |
| E | EP 2 123 626 A1 (ESTEVE LABOR DR [ES])<br>25 November 2009 (2009-11-25)<br>* the whole document *<br>----- | 1-15 | |

The present search report has been drawn up for all claims

3

| Place of search | Date of completion of the search | Examiner |
|---|---|---|
| Munich | 19 March 2010 | Lauro, Paola |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 09 38 4006

This annex lists the patent family members relating to the patent documents cited in the above-mentioned European search report.
The members are as contained in the European Patent Office EDP file on
The European Patent Office is in no way liable for these particulars which are merely given for the purpose of information.

19-03-2010

| Patent document cited in search report | Publication date | Patent family member(s) | Publication date |
|---|---|---|---|
| WO 0028980 A2 | 25-05-2000 | AT 234090 T | 15-03-2003 |
| | | AU 1718400 A | 05-06-2000 |
| | | CA 2350531 A1 | 25-05-2000 |
| | | DE 69905938 D1 | 17-04-2003 |
| | | DE 69905938 T2 | 13-11-2003 |
| | | EP 1135121 A2 | 26-09-2001 |
| | | ES 2194536 T3 | 16-11-2003 |
| | | JP 2002529499 T | 10-09-2002 |
| WO 2007134168 A2 | 22-11-2007 | EP 2016066 A2 | 21-01-2009 |
| WO 2008133884 A2 | 06-11-2008 | NONE | |
| EP 2123626 A1 | 25-11-2009 | WO 2009141144 A1 | 26-11-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Pharmaceutics: The Science of Dosage Forms. 2002
- Encyclopedia of Pharmaceutical Technology. Marcel Dekker, Inc, 2002
- Modern Pharmaceutics. Marcel Dekker, Inc, 2002
- The Theory and Practice of Industrial Pharmacy. Lea & Febiger, 1986
- Modified-Release Drug Delivery Technology. Marcel Dekker, Inc, 2002
- Handbook of Pharmaceutical Controlled Release Technology. Marcel Dekker, Inc, 2000
- Controlled Drug Delivery. Basic Concepts. CRD Press Inc, 1983, vol. I
- Oral Drug Delivery. **TAKADA, K. ; YOSHIKAWA, H.** Encyclopedia of Controlled Drug Delivery. John Wiley & Sons, Inc, 1999, vol. 2, 728-742
- Oral drug delivery, small intestine and colon. **FIX, J.** Encyclopedia of Controlled Drug Delivery. John Wiley & Sons, Inc, 1999, vol. 2, 698-728